(19) Europäisches Patentamt
European Patent Office
Office européen des brevets

(11) EP 4 181 781 B1

(12) # EUROPEAN PATENT SPECIFICATION

(45) Date of publication and mention of the grant of the patent:
**20.05.2026 Bulletin 2026/21**

(21) Application number: **21745996.5**

(22) Date of filing: **16.07.2021**

(51) International Patent Classification (IPC):
***A61B 5/08*** *(2006.01)*

(52) Cooperative Patent Classification (CPC):
**A61B 5/082; A61B 5/4845**

(86) International application number:
**PCT/EP2021/069925**

(87) International publication number:
**WO 2022/013413 (20.01.2022 Gazette 2022/03)**

(54) **BREATH-BASED THERAPEUTIC DRUG MONITORING METHOD**

ATEMBASIERTES VERFAHREN ZUR ÜBERWACHUNG VON THERAPEUTISCHEN ARZNEIMITTELN

PROCÉDÉ DE PHARMACOVIGILANCE THÉRAPEUTIQUE BASÉ SUR LA RESPIRATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.07.2020 EP 20186274**

(43) Date of publication of application:
**24.05.2023 Bulletin 2023/21**

(73) Proprietors:
- **Universitäts-Kinderspital beider Basel**
  **4056 Basel (CH)**
- **Universität Zürich**
  **8006 Zürich (CH)**

(72) Inventors:
- **SINGH, Kapil Dev**
  **4056 Basel (CH)**
- **GISLER, Amanda**
  **4056 Basel (CH)**
- **OSSWALD, Martin**
  **8091 Zürich (CH)**
- **KOHLER, Malcolm**
  **8091 Zürich (CH)**
- **VAN DEN ANKER, Johannes**
  **4056 Basel (CH)**
- **VON DER WEID, Nicolas**
  **4056 Basel (CH)**
- **FREY, Urs**
  **4056 Basel (CH)**
- **DATTA, Alexandre**
  **4056 Basel (CH)**
- **SINUES, Pablo**
  **4056 Basel (CH)**

(74) Representative: **Vossius & Partner**
**Patentanwälte Rechtsanwälte mbB**
**Siebertstraße 3**
**81675 München (DE)**

(56) References cited:
**WO-A2-2014/117747 US-A1- 2012 016 252**

- **YUICHI SAKUMURA ET AL: "Diagnosis by Volatile Organic Compounds in Exhaled Breath from Lung Cancer Patients Using Support Vector Machine Algorithm", SENSORS, vol. 17, no. 2, 4 February 2017 (2017-02-04), pages 287, XP055692117, DOI: 10.3390/s17020287**
- **CHRIS O PHILLIPS ET AL: "Machine learning methods on exhaled volatile organic compounds for distinguishing COPD patients from healthy controls", JOURNAL OF BREATH RESEARCH, vol. 6, no. 3, 4 July 2012 (2012-07-04), US, pages 036003, XP055692114, ISSN: 1752-7155, DOI: 10.1088/1752-7155/6/3/036003**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

• **SKARYSZ ANGELIKA ET AL: "Convolutional neural networks for automated targeted analysis of raw gas chromatography-mass spectrometry data", 2018 INTERNATIONAL JOINT CONFERENCE ON NEURAL NETWORKS (IJCNN), IEEE, 8 July 2018 (2018-07-08), pages 1 - 8, XP033419390, DOI: 10.1109/IJCNN.2018.8489539**

## Description

The invention relates to methods for therapeutic drug monitoring and apparatus for use in said methods.

The concept of personalized medicine revolves around the idea of providing the right treatment to the right person at the right time. In this context, therapeutic management of chronically ill patients represents a major challenge in routine clinical practice. Similarly, in the drug development pipelines, patient's therapeutic response and manifestation of unwanted side effects often proves to be highly patient-specific. Thus, in the absence of objective surrogates, empiric therapy is often the only available option. However, highly-individual specific response to therapy, driven by genetic background, environment and ultimately patient-specific metabolism, makes this approach suboptimal. One common approach to optimize drug dosage, both during clinical trials and also when the drugs are already in the market, is to determine drug concentrations in plasma/serum and adjust the dosage accordingly to keep the concentrations within the so-called therapeutic range. Thus, the purpose of therapeutic drug monitoring (TDM) is individualizing the dose to achieve maximum efficacy and, at the same time, minimize toxicity. TDM has proven to be a valuable strategy towards a more tailored pharmacotherapy, ultimately resulting in improved patient outcome and minimized side effects in multiple conditions.

Therapeutic drug monitoring is typically performed by the analysis of blood samples provided from a subject for determining the concentration of a particular chemical substance. Non-limiting examples of techniques useful for this purpose include immunoassays and gas or liquid chromatography coupled to mass spectrometry (GCMS or LCMS). It will be noted that these techniques are typically optimized for direct determination of the presence and/or concentration of a single selected chemical substance in a subject. In addition, existing TDM techniques usually require invasive steps, e.g. for obtaining a blood or tissue sample from a subject.

Currently available non-invasive methods are far from being able to replace such invasive methods due to limitations in reliability, specificity and the like.

TDM has limitations. Firstly, in its typical embodiment it relies on venipunctures (i.e. accessing samples of venal blood of a subject) to determine drug concentrations, which are not well tolerated by children. Secondly, drug concentrations often do not correlate with improved clinical outcome and minimized side effects, due to highly patient-specific drug metabolism [Kang & Lee 2009].

One condition whereby such limitations are often manifested is epilepsy. Epilepsy is a complex neurological disorder affecting around 50 million people worldwide caused by the insult to the brain or genetic mutations [Beghi et al. 2019]. However, treatment with one or more antiepileptic drugs (AEDs) can allow roughly 70% of patients to live seizure free, but in the long run, 40% of those patients relapse and about 25% develops resistance against therapy [Schiller 2009]. As a result, the overall therapeutic management of epilepsy, despite using TDM methods, remains a challenge. The same complex situation emerges in other diseases requiring medication with narrow therapeutic ranges such as cancer treatment using chemotherapy, antidepressants, antibiotics and immunosuppressive drugs [Dasgupta 2012]. Such individualized response to medication with narrow therapeutic ranges calls for a more comprehensive phenotyping approach, beyond just monitoring of systemic drug concentrations. In this regard, pharmacometabolomics has been proposed as a tool to better predict individual drug pharmacokinetic and pharmacodynamic characteristics. Accordingly, the purpose of this invention is to provide a means of detecting drugs, as well as drug-regulated metabolites in exhaled breath, to better phenotype patients requiring TDM or in clinical trials during drug development programs, leading to a more tailored therapeutic management. Relevant prior art is disclosed in US 2012/0016252 A1, which provides a method for therapeutic drug monitoring by analysing exhaled breath using mass spectrometry to determine drug concentration or its drug-derived metabolites in the body.

The underlying technical problem is thus the provision of methods for determining the concentration of a chemical substance in a subject by a non-invasive method in a reliable matter. The technical problem is solved by embodiments provided herein and as characterized in the claims.

In a first aspect, the present disclosure relates to an *ex vivo* method for determining the concentration of a chemical substance in a subject, the method comprising: (a) providing a sample of exhaled breath by the subject to a mass spectrometer; (b) collecting the mass spectra of substances present in the exhaled breath in positive and/or negative mode; (c) analysing the mass spectra using a previously trained mathematical model; and (d) determining the concentration of the chemical substance in the subject based on the analysis of the mass spectra of the exhaled breath; wherein the previously trained mathematical model relies on the signal intensity or area under the curve of selected m/z peaks in the mass spectra as predictors.

The analysis of chemical substances in a subject based on the analysis of respiration (breath) allows for an insight into the metabolism of the subject on a non-invasive basis. Traditional methods for determining the concentration of chemical substances are based on the direct detection of the substance of interest or one or more of the immediate metabolites. As such, methods of the prior art rely on pre-defined direct and/or indirect markers of the presence of a chemical substance of interest. The methods of the present invention may rely on such markers, however, in contrast to the

methods of the prior art, the present invention relates to a holistic approach to determining the concentration of a chemical substance and/or monitoring concentration of a drug in a subject. In this regard, the present inventors have surprisingly found that mass spectrometry measurements of a sample of exhaled breath of a subject, the sample in particular comprising exhaled metabolites, can be used to determine the concentration of a chemical substance in said subject, based on the analysis of the spectra using a previously trained mathematical model. The volatile compound or compounds may be related to the said chemical substance, i.e. may be metabolite(s) of said substance. However, the inventors have surprisingly found that effects dependent on the concentration of said chemical substance on the level of volatile compounds that are drug-regulated metabolites, may alternatively or additionally be used to determine the concentration of said chemical substance, without prior knowledge of such effects. The inventors have further surprisingly found that effects dependent on the concentration of said chemical substance on the level of unrelated volatile compounds (i.e. compounds that are not metabolites of said chemical substance) may be used to determine therapeutic or toxic effects (e.g., probability estimate of drug response and/or risk estimate of side effects of a chemical substance) of said chemical substance.

As demonstrated in the Examples, the method of the present invention does not require that the chemical substance is a volatile component of blood, and/or can be observed directly in a sample of breath exhaled by a subject using mass spectrometry (as exemplified in Example 7). Instead, in the methods of the present invention, it is possible to determine the concentration of a chemical substance based on changes in abundances or levels of metabolites of the chemical substance or changes in abundances or levels of other metabolites. That is, it is only required that the composition of the exhaled breath is modulated upon presence of the chemical substance. As such, the methods of the present invention provide means for determining the concentration of a chemical substance in a subject without prior knowledge of how the said composition is affected by the said substance.

As defined herein, the concentration of a chemical substance in a subject preferably refers to the serum concentration of a chemical substance in a subject. It is noted that both total serum concentration of a chemical substance as well as free serum concentration of a chemical substance can be determined using the methods of the present invention. The free serum concentration of a chemical substance is defined preferably as the concentration of the chemical substance that is not bound to any proteins. In certain embodiments of the present invention, determining the concentration of a chemical substance in a subject may be limited to determining whether said concentration is higher or lower than a certain threshold value. Depending on the said threshold value, in certain embodiments of the present invention determining the concentration of a chemical substance in a subject may thus be understood as determining the presence of absence of the chemical substance in the subject, wherein the presence of the chemical substance in the subject is understood that the concentration of the chemical substance in the subject is not less than the said threshold value.

Breath herein is defined as a bodily fluid exhaled from lungs. Breath is in a gaseous form and comprises nitrogen typically in an amount of between 70% and 75% of breath by volume, oxygen typically in an amount of between 13% and 16% of breath by volume, carbon dioxide typically in an amount of between 5% and 6% of breath by volume, water vapour typically in an amount of between 4% and 6% of breath by volume, and argon typically in an amount of 1% of breath by volume. Other substances maybe present in breath in an amount of less than 0.01 % of breath by volume. In particular, organic compounds that originate from a subject are present in the breath. As it will be noted herein, the organic compounds, or the metabolites, that are present in the breath, are referred to as a breath metabolome, or volatilome.

The subject as understood herein is preferably a human subject. However, the embodiments of this invention may also refer to a subject that is non-human animal, provided that collection of samples of exhaled breath from the subject can be performed. In the methods of the present invention, the subject investigated is preferably a patient receiving medication, either an approved drug or combination of drugs, or an experimental drug during a clinical trial. More preferably, the patient is a patient receiving medication in need of TDM.

Mass spectrometry is an analytical technique that measures the mass-to-charge ratio and relative intensity for ions that are sampled into a mass spectrometer. The mass-to-charge ratio is also referred to as m/z parameter, wherein m is the molecular mass of said ion, preferably expressed in Daltons, and z is its charge. Herein, the charge z is typically described by its absolute value, ignoring its sign. The results of such measurement for a sample are typically presented as a mass spectrum, wherein on the x axis the m/z parameter is shown, and on the y axis the intensity of the measured signal is shown. The signal intensity is proportional to the number of ions of a particular m/z observed in the measurement, and is a measure of the number of ions of a particular m/z sampled to a mass spectrometer. The said number of ions may also be referred to as abundance of ions of a particular m/z. Therefore, peaks in the mass spectrum are characterized by their m/z parameter (also referred to as m/z ratio), and the intensity or the area under curve, which are dependent on the number of ions of a particular m/z observed during measurement. Performing mass spectrometry measurements on a sample will herein also be referred to as collecting a mass spectrum of a sample.

In the methods of the present invention, a mass spectrometer is used for collecting mass spectra of breath samples. In other words, preferably the mass spectrometer is previously calibrated for analysis of breath samples. Preferably, a high resolution (i.e. Resolution > 10,000, as defined by IUPAC) atmospheric pressure ionization mass spectrometer is used in the method of the present invention. Non-limiting examples of such a mass spectrometers include

the Sciex's TripleTOF and Thermo's Q-Exactive Plus Mass Spectrometer.

Emerging analytical technologies such as secondary electrospray ionization-high resolution mass spectrometry (SESI-HRMS) have enabled collecting mass spectra of breath samples over the last decade. Breath metabolome analysis by SESI-HRMS is non-invasive technique (i.e. no need for venipuctures for the collection of blood of a subject) that is faster than other metabolome analysis techniques. Collecting mass spectra of breath metabolome allows performing actual compound identification of the detected molecules, which is not possible with other techniques, for examples using sensors). Ability to identify compounds in the breath metabolome allows providing biochemical interpretations of metabolism at the molecular level, hence it allows insights into the pathophysiology and drug-disease interplay. SESI-HRMS has developed to a standardized technique applicable in clinical settings [Singh et al. 2018; Singh et al. 2019]. One of the present inventors herein have used a slight variant of this approach in U.S. patent applications Ser. No. 11/732,770 and 12/556,247.

Herein, the metabolome is understood as comprising low molecular weight compounds that are produced in the cell, or more generally, the organism, as a result of its life processes. These compounds are referred to as metabolites. The term low molecular weight compounds refers to the compounds with molecular weight of preferably less than 1000 Da. It is known to the person skilled in the art that different portions of the metabolome will be present, or observable, in different bodily fluids. The breath metabolome refers to the portion of the metabolome that is comprised in breath. The term "metabolites" refers to intermediate or end products of metabolism, typically small molecule organic compounds. In other words, metabolites are the molecules encompassed within the metabolome.

Metabolism is defined as a set of chemical reactions, typically catalysed by enzymes present in an organism that are necessary for sustaining of life. These processes include conversion of external nutrients to energy required to run cellular processes, and/or the conversion of nutrients or energy sources to building blocks for proteins, lipids, nucleic acids, and/or carbohydrates, and/or the elimination of cellular waste. The chemical reactions of metabolism can form reaction cascades in such a way that a product of one reaction is a substrate of another reaction. Two or more reactions wherein a product of one reaction is a substrate of another reaction are also referred to as reaction cascades. Such reaction cascades are also referred to as metabolic pathways.

The process of collecting mass spectra is known to the person skilled in the art. It is further noted that the person skilled in the art will understand that different modes of collecting the mass spectra are used for detection of positively charged ions and of negatively charged ions.

These two different modes of collecting the mass spectra are also referred to as collecting spectra in a positive mode, or a positive collection mode, and as collecting spectra in a negative mode, or negative collection mode, respectively. It is known to the person skilled in the art that the positive collection mode is configured for detection of positively charged ions, and that the negative collection mode is configured for detection of negatively charged ions. Herein, a positive collection mode will be referred to as a positive mode, and a negative collection mode will be referred to as a negative mode.

A sample of breath exhaled by a subject, also referred to as a breath sample, can be provided to the mass spectrometer, or in other words sampled to the mass spectrometer, in several different ways. Preferably, samples of exhaled breath are provided by a subject directly to the mass spectrometer, by exhaling breath directly into the mass spectrometer apparatus, preferably configured for provision of exhaled breath samples. However, this setup is not limiting and this invention also relates to embodiments, wherein breath samples are collected into gas containers configured for collection and storage of breath exhaled by a subject, preferably sealed and/or attached to the mass spectrometer, preferably configured for provision of gas samples, preferably exhaled breath samples.

In the methods of the present invention, mass spectra of substances comprised in samples of exhaled breath are collected. Mass spectrometry allows simultaneous observation of several species present in the sample by collecting the mass spectrum, herein in the exhaled breath. Exhaled breath contains several substances that may, inter alia, be due to metabolic processes or other processes resulting in volatile compounds comprised in exhaled breath that can be detected in the gas phase. The substances that can be detected in the exhaled breath in the gas phase, preferably including metabolites, are also referred to as volatilome.

In order to be detectable by mass spectrometry, molecules need to bear a charge. Therefore, compounds provided for mass spectrometry measurements, or sampled to a mass spectrometer, are transformed into chemical entities that comprise a charge - ions. This process is referred to as ionisation. Several methods of ionisation of compounds for analysis by the mass spectrometry are known to the person skilled in the art.

Electrospray ionization is one method useful for transforming compounds into ions for analysis by the mass spectrometry. In this method, high-voltage is applied to a liquid solution comprising a compound to create an aerosol. Aerosol is herein defined as droplets of liquid dispersed in a gas phase. The liquid droplets in the aerosol lose solvent, leaving charged particles of the compound in the gas phase. Electrospray ionization can produce ions that are multiply charged. Matrix-assisted laser desorption/ionization, also referred to as MALDI, is another method useful in transforming chemical compounds into ions for mass spectrometry analysis. In this method, a chemical compound is mixed with a matrix material and then irradiated with laser pulses, which leads to desorption of the chemical compound. The chemical

compound then undergoes protonation or deprotonation in the ablated gases and the so-obtained ions derived from the chemical compound can be subjected to mass spectrometry analysis. Typically, MALDI produces ions that preferably are singly charged.

Secondary Electrospray Ionisation, also referred to as SESI, is an ionization setup that is suitable for ionisation of exhaled breath samples before subjecting them to the mass spectrometry analysis. In this approach, the electrospray ionisation is first used to produce ions in the gas phase, also referred to as charging ions. The charging ions are then contacted with the chemical compound that is to be ionised. Non-limiting examples of substances suitable for the use in such electrospray to produce charging ions are water and formic acid.

Any ionisation method that is suitable for transforming gaseous samples into ions before they are subjected to the mass spectrometry analysis can be used in the methods of the present invention. Preferably, secondary electro spray ionization, also referred to as SESI, is used in the methods of the present invention. A preferred solution for use in the secondary electro spray ionization is water solution comprising 0.1% of formic acid. However, this composition is not limiting and a variety of other solutions can be used in the said method.

An alternative embodiment uses a plasma ion source interfaced to the mass spectrometer. The ionization method involves a high-frequency cold plasma, inducing few in-source fragmentation [Bregy et al, 2014].

Figure 1 illustrates an exemplary embodiment , whereby a subject exhales into a SESI ionizer through a disposable mouthpiece. Any mass spectrometer suitable for collecting spectra of gaseous samples, preferably samples of exhaled breath, can be used in the method of the present invention. Preferably, a mass spectrometer useful in the method of the present invention is a high-resolution mass spectrometer, also referred to as HRMS. A high-resolution mass spectrometer is defined as a mass spectrometer, which is capable to detect substances present in the gaseous composition at a concentration of 1 part per billion (1 ppb) or less, or preferably at a concentration of 10 part per trillion (ppt) or less, or most preferably at a concentration of 1 ppt or less. Preferably, the invention uses a SESI ionizer interfaced to an atmospheric pressure interface mass spectrometer (preferably high resolution mass spectrometer configured for breath analysis). A non limiting example of a mass spectrometer suitable for such application is Orbitrap HRMS. A further non limiting example of a mass spectrometer within Orbitrap HRMS family suitable for such application is Q Exactive Plus Mass Spectrometer.

Herein, a mouthpiece is defined as a hollow object with an opening of both sides. One side of the mouthpiece is preferably configured to be attached to the mouth of a subject to allow for a tight flow of exhaled breath into the mouthpiece. The other side of the mouthpiece is configured to preferably be attached to a connector for delivering the collected sample to an ionisation device, so that the flow of exhaled breath from the mouth of the subject, preferably through the mouthpiece, can be directed into the connector and then to the ionization device. Optionally, within the opening of the mouthpiece a filter may be placed and/or configured in such a way, that the breath exhaled through the mouthpiece flows through the filter. Any mouthpiece that fulfils these requirements and/or is suitable for directing a sample of breath exhaled by a subject into the connector can be used in the methods of the present invention and with the apparatus of the present invention. For a non-limiting example, any mouthpiece known to the person skilled in the art to be suitable as mouthpiece for spirometry measurement, can be used in the methods and with the apparatus of the present invention.

The mass spectra collected from the sample of exhaled breath will contain information on the chemical compound(s) comprised in the sample. In the process of mass spectrometry measurements, chemical compounds are transformed into ions, and each ion gives raise to a peak or peaks. Peaks in the mass spectrum are characterized by m/z ratio, wherein m is the mass of detected ion, z is its charge, and the intensity of a peak or the integrated area under the peak, also referred to as area under the curve, corresponds to the amount (or abundance) of a chemical compound that is detected by the mass spectrometer. Preferably, the area under the curve parameter is used. In other words, mass spectrometry data is represented in the form of m/z peaks. Each of the peaks is represented by its m/z ratio, which is indicative of the type of chemical species the said peak is due to, and by its intensity or area under the curve, which is indicative of the abundance of the species of particular m/z. Area under the curve parameters are normalized to allow comparisons between different measurements, yielding a normalised area under the curve parameter for each peak, also referred to as an nAUC parameter. The integrated area under the curve (AUC) for all features is normalized by the exhaled volume in the exhalation maneuver. Exhaled volume can be estimated by measuring exhaled flow rate via pressure drop in a flow-calibrated restricted tube and exhalation time. A non-limiting example of such device suitable for such application is Exhalion (FossilIonTech, Spain), depicted in Figure 1 as a black box with touch screen. Presence of a peak of a particular m/z in the spectrum is a sufficient proof that a chemical compound of a mass that could give raise to a said peak upon being transformed to a detected ion is present in the exhaled breath. Multiple peaks due to different ions are typically detected in a mass spectrum. In the method of the present invention, the concentration of a chemical substance in a subject is determined based on the analysis of a mass spectrum/a collected from a sample(s) of breath exhaled by the subject. Herein, analysis of mass spectra comprises a holistic analysis that takes into account all or a majority of the peaks observed in the mass spectrum. It will be noted that the said peaks do not need to be due to ions originated from the said chemical substance or the metabolite thereof.

The high-resolution mass spectra of substances present in exhaled breath obtained during the exhalation by

patients according to the method of the present invention may comprise hundreds of mass spectral features. In the method of present invention, only a number of features, previously identified as relevant features, are used to compute i) concentration of a chemical substance in a subject, preferably a systemic drug concentration; and/or ii) probability of suffering of side effects, also referred to as risk estimate of side effects; and/or iii) probability that the patient is responding to the pharmacotherapy properly, also referred to as probability of drug response.

In the methods of the present invention, the mass spectra are analysed using a previously trained mathematical model. The previously trained mathematical model relies on predictors that are selected from available mass spectrometry data, in other words from all or at least the majority of the peaks present in the mass spectra. The term "predictor" is herein understood as a measurable variable that is usable as input in a previously trained mathematical model. It should be noted that a predictor, or a predictor variable, is preferably an independent variable, that means a variable that cannot be represented as a combination of other variables. Predictor variables, also known as independent variables, can be obtained by subjecting all the available variables to a feature reduction analysis. Herein, the predictors selected from mass spectrometry data are preferably peaks in the mass spectrum, characterized by their m/z parameter and either their signal intensity or, preferably, normalised area under the curve (nAUC) parameter. Herein, for a predictor corresponding to a particular m/z peak, its signal intensity or preferably nAUC parameter may also be referred to as a value of the predictor.

To select predictors from all the available data, preferably a training dataset is required, which contains not only the information on the collected mass spectra of samples of breath exhaled by subjects (breath samples), but preferably also information on the concentrations of the chemical substance in subjects, preferably as determined from the blood of the subject by using approaches known to a person skilled in the art, including immunoassays, GC-MS or LC-MS.. In other words, the feature selection process is preferably done using a pre-existing (i.e. training) dataset of collected mass spectra of breath exhaled by subjects receiving the same medication(s) and for whom the systemic drug concentrations have been previously determined using methods known to the skilled person. For preferably each subject included in the training set, a response vector that comprises the variables to be extracted from the peaks in the mass spectra, i.e. description of observed peaks in the mass spectra, as well as a target value. is constructed. Herein, the target value may be the concentration of the chemical substance in the subject. However, the target value may also refer to other clinical parameters, for example relating to side effects and/or drug response. To select predictors, the response vectors constructed for each subject are combined into a matrix and subjected to feature reduction analysis. Herein, the terms feature selection and feature reduction analysis are used interchangeably.

The training dataset is not limited to comprise the systemic drug concentration for each subject as a target value. Herein, the systemic drug concentration is understood as previously determined using methods known to the skilled person, for example from the blood of the subject. In the case of the prediction of systemic drug concentration, the target variable will be a numeric value. A target value may also include further clinically relevant parameter(s), including data relating to drug response and/or side effects, also referred to as therapeutic effects. Side effects and drug response data for inclusion in the dataset are preferably determined by trained clinicians using standard and objective clinical endpoints, known to the person skilled in the art. Such a therapeutic effect can be represented by a numeric value or a categorical value. Non-limiting examples of numerical and categorical values include reduction of number of seizures (for example reduction of seizures observed upon administration of antiepileptic drugs - AEDs) or cancer remission (for example cancer remission as observed after chemotherapy), respectively. Similarly, side effects can be described by numeric or categorical variables assessing a broad range of conditions. Non-limiting examples of such conditions are somnolence, nausea or vomiting, stomach pain or upset, diarrhoea, hair loss, tiredness, dizziness, chills, and headache. It is noted that the methods of the present invention are applicable both when the target value comprises a numerical value, as well as when the target value comprises a categorical value.

It is known to the person skilled in the art that a feature reduction analysis may result in selecting independent variables from a set of all the variables, which can be used as predictors as defined herein. As a result of the feature reduction analysis performed on the matrix containing all the response vectors, a reduced response vector matrix containing smaller number of variables is constructed. The mass spectrometry data included in thus-constructed response vector are independent variables and are referred to as predictors. The so-selected set of predictors is then used to train a mathematical model to correctly determine the presence of the chemical substance in the subject and/or the concentration of the chemical substance in the subject.

The methods of feature reduction analysis are intended to reduce the number of input variables to those that are the most useful in a mathematical model for determining, or predicting, the target variable. Such input variables selected in the course of the feature reduction analysis are referred to as predictors. There are two main types of the methods of the feature selection analysis: wrapper methods and filter methods. Any of both approaches is useful in identifying the most relevant mass spectral features, in other words the m/z peaks in spectra collected according to the methods of the present invention, also referred to as the input variables to be used as predictors.

Filter methods are feature selection methods based on statistical analysis. These methods rely only on characteristics of features and no machine learning method or algorithm is used herein. Filter methods are useful in eliminating features that are irrelevant, duplicated, correlated with each other, and/or redundant. There are univariate and

multivariate filter methods. Univariate filter methods evaluate and rank each single feature according to defined criteria, and then allow selection of features with the highest ranking according to the said defined criteria. Univariate filter methods do not consider correlations between features, therefore duplicate and/or redundant features may be selected in this approach. Multivariate filter methods in turn evaluate more features simultaneously, at least two features simultaneously, and are capable of dealing with the problem of duplicate and/or redundant features. Filter methods include, but are not limited to, Pearson correlation coefficient, Spearman's rank correlation coefficient, Kendall's rank correlation coefficient, Chi-squared score, Analysis of variance test, ROC-AUC/RMSE.

Wrapper methods are feature selection methods based on a machine learning algorithm. Given the machine learning algorithm of choice, wrapper methods explore of different subsets of variables and evaluate each subset for performance of the said algorithm. Exploration of different subsets of variables is done according to an employed search strategy. Any search strategy and any machine learning algorithm is suitable for use in the wrapper methods. Wrapper methods can detect correlation between variables and can find optimal feature subset for the machine learning algorithm or method of choice. Typically, wrapper methods are more computationally expensive than filter methods, but can provide a subset of features that perform better in the selected machine learning algorithm or method than a subset of features selected by using filter methods. The search strategies for use in the wrapper methods include forward feature selection, backward feature elimination, exhaustive feature selection and bidirectional search. In the forward feature selection, the search starts with no feature and one is added at a time. In each cycle, a feature that provides the best performance is selected and retained in the feature subset. In the backward feature elimination, the search starts with all the available features and one is removed at a time. In the exhaustive feature selection, all the possible combinations of features are evaluated. In the bidirectional elimination, forward feature selection and backward feature elimination are performed simultaneously and combined to achieve the best possible result. To assure that both methods converge to the same solution, restrains are introduced wherein a feature added by forward feature selection cannot be removed by backward feature elimination, and wherein a feature removed by backward feature elimination cannot be added by forward feature selection.

The so-obtained set of predictors is used to train a mathematical model to correctly predict the concentration of the chemical substance in subjects. Any trainable mathematical model, preferably a regression method, can be used in the methods of the present invention. Preferably, any machine learning regression algorithm can be used in the methods of the present invention. More preferably, a supervised machine learning regression algorithm can be used in the methods of the present invention. Such algorithms include, but are not limited to, decision tree algorithms, support vector machines, and Gaussian process regression algorithms.

A decision tree model, also referred to as decision tree is a predictive modelling approach to go from observations about an item to conclusions about the item's target value. Decision tree models where the target variable takes a discrete set of values are called classification trees, while decision trees where the target variable can take continuous values, e.g. real numbers, are called regression trees. Decision tree models include, but are not limited to, fine tree, medium tree, coarse tree, boosted tree, bagged tree.

Support vector machines are supervised learning models associated with learning algorithms that analyse data used for classification and regression analysis. A support vector machine, also referred to as support vector model or SVM, is a representation of the examples as points in space, mapped so that the examples of the separate categories are divided by a clear gap that is as wide as possible. New examples are then mapped into that same space and predicted to belong to a category based on the side of the gap on which they fall. Support vector machines include, but are not limited to, linear SVM, quadratic SVM, cubic SVM, fine granular SVM, medium granular SVM, coarse granular SVM.

The mathematical model used in the present invention is preferably a Gaussian process regression (GPR). A Gaussian process is a stochastic process comprising a collection of random variables indexed preferably by time or space, such that every finite collection of those random variables has a multivariate normal distribution. GPR is a nonparametric (i.e. does not require that the population being analyzed meet certain assumptions, or parameters), Bayesian approach (i.e. a probabilistic construct that allows new information to be combined with existing information) to regression that infers a probability distribution over all possible values.

Consider the training set $\{(x_i, x_i); i = 1,2, ..., n\}$, where $x_i \in \mathbb{R}^d$ *and* $y_i \in \mathbb{R}$, drawn from an unknown distribution. A GPR model addresses the question of predicting the value of a response variable $y_{new}$, given the new input vector $x_{new}$, and the training data. A linear regression model is of the form $y = x^T\beta + \varepsilon$, where $\varepsilon \sim N(0, \sigma^2)$. The error variance $\sigma^2$ and the coefficients $\beta$ are estimated from the data. A GPR model explains the response by introducing variables called the latent variables, $f(x_i)$, $i = 1,2, ... , n$ from a Gaussian process, and explicit basis functions, $h$. The covariance function of the latent variables captures the smoothness of the response and basis functions project the inputs $x$ into a $p$-dimensional feature space. Hence, a GPR model is a probabilistic model. There is a latent variable $f(x_i)$ introduced for each observation $x_i$, which makes the GPR model nonparametric.

The so-trained regression method can rely on predictors, that means input variables, extracted from the collected mass spectra of substances present in the samples of exhaled breath from subjects, the spectra that were not used in the

training of the mathematical model of the present invention, for determining the concentration of the chemical substance in a subject.

Figure 2 presents an exemplary analysis pipeline that is used to select predictors based on the analysis of mass spectra of exhaled breath by subjects. The subjects may be a user or non-user of a substance. Based on the analysis of mass spectra of samples of exhaled breath, preferably for each subject a response vector is constructed that includes nAUC parameters for m/z peaks observed in the said spectra as well as the concentration of the chemical substance in the subject. For an embodiment presented in Figure 2, 385 variables were considered. These variables correspond to peaks in the collected mass spectra. Herein, the chemical substance is valproate. For subjects that are users of valproate, the concentration is given as the measured serum concentration of valproate, preferably as measured using the methods that form state of the art, including analysis of blood of a subject, while for non-users the concentration has been assigned as zero. Based on the feature selection analysis, the number of variables is reduced to 12, which are also referred to as independent variables, predictor variables or predictors, and the regression method is selected and trained thereon. Herein, the 12 predictors correspond to 12 peaks in the mass spectra, due to the drug itself, herein valproate, and three metabolites thereof.

Thus, the method of the present invention preferably uses complete information on the composition of the volatilome to determine the concentration of a chemical substance in a subject. Such an approach is likely to be more accurate than approaches known to the person skilled in the art, for example determination of the concentration of particular single substances in blood of a subject. As demonstrated in Example 3 and Reference Example 3, the method of the present invention wherein the previously trained mathematical model relies on 12 predictors, outperforms the determination of the concentration of valproate in a subject relying on a single m/z peak, due to particular metabolite (as demonstrated both for 3-heptanone and 2-propyl-4-pentanolactone).

It will also be noted that the values (i.e. signal intensities of nAUC) of predictors corresponding to m/z peaks due to 3-heptanone and 2-propyl-4-pentanolactone vary significantly between the subjects, and also vary significantly for each particular subject with time, as demonstrated in Example 2 and illustrated in Figure 3. It is known to the person skilled in the art that the way a particular drug is metabolized varies significantly across different individuals or even within-individuals over time. The methods of the present invention do not rely on the presence or abundance or level of any single selected component of the volatilome. Instead, the methods of the present invention allow integration of information on the composition of the volatilome. As such, the contribution of isolated components is reduced and effects as described above are less severe. If a method that predicates on detection of a single component of the volatilome that is variable between the subjects, was used herein, it would lead to false determination of a concentration of the chemical substance in cases wherein variation of the level of a particular single component of the volatilome is due to individual characteristics of the subject and not due to the presence of a chemical substance in the subject. It was surprisingly found that analysis using the methods provided herein are more reliable and less impacted by natural variation of the activity of different metabolic pathways. It was further surprisingly found that there is a variability within- one subject and between-subjects of the said ratios of signal intensity of the predictors, allowing to gain access to individual differences in the metabolism of VPA.

In one embodiment , the mass spectrum, preferably obtained by using SESI-HRMS, collected from substances present in the samples of exhaled breath, in other words collected from human breath, produces a rich and readily interpretable pattern of ions dominated by drugs, drug-related metabolites, as well as endogenous metabolites (including drug-regulated metabolites). The resulting complex but clear series of pairs of numbers (ion mass-to-charge ratio and signal intensity) is rapidly interpreted by: i) comparison of drug-related ion abundances to previously known breath mass spectra of subjects with known systemic drug concentrations and/or ii) comparison of endogenous metabolites' ion abundances to previously known breath mass spectra of subjects with known response to medication, pharmacokinetic and pharmacodynamic parameters that best correlate with clinical outcome, and/or unwanted side effects.

Within the present invention, for providing a sample of breath exhaled by the subject to a mass spectrometer, the subject preferably exhales into a mouthpiece attached to a connector for delivering the collected sample to the ionization device, for example as shown in Figure 1. The exhalation maneuver is performed preferably through a disposable bacterial and/or viral filter. A typical breath test preferably requires parallel monitoring of the exhalation flow rate, exhalation volume and carbon dioxide content with the aim of standardizing the exhalation maneuver. During each measurement, the subject preferably provides 5-6 replicate exhalations both in a positive and in a negative ion mode. Figure 4 illustrates a resulting total ion current (also referred to as TIC) in positive ion mode during such exhalation maneuvers, whereby the signal intensity rises above the background level (1) during the exhalation maneuvers (2). Preferably, the background levels are obtained from clean dry nitrogen flushed continuously through the ion source when the subject is not exhaling.

Herein, the total ion current (TIC) refers to the sum of the relative abundances of all the observed ions in the mass spectrum at a selected time interval, or during a selected scan. Typically, the total ion current is plotted against time, or against scan number.

In a further aspect, the methods of the present disclosure relate to an embodiment, wherein the mass spectrometer is calibrated for analysis of breath samples prior to step (a). In a more preferred embodiment, the methods of the present disclosure relate to an embodiment, wherein the mass spectrometer is calibrated by providing the mass

spectrometer with a standard gas mixture and monitoring the stability of the signal.

A preferred procedure prior to collecting the mass spectra of substances present in the exhaled breath in a positive and/or a negative mode, in particular for calibrating the mass spectrometer, involves the calibration and optimization of the mass spectrometer for the detection of gas species with a standard gas mixture. The standard gas mixture preferably comprises substances that behave similarly in the mass spectrometry setup for breath sample analysis as the samples of exhaled breath. That means, the standard gas mixture preferably comprises substances with masses of 50-300 Da. Non-limiting examples of substances that are useful as components of the standard gas preferably include acetone, isoprene, 2-butanone, 2-pentanone, styrene, mesitylene, and/or terpene, preferably at concentrations ranging from 1 ppt to 10 ppb.

The time series of the signal intensity of the individual compounds, or linear combinations thereof, are evaluated following preferably, but non-limiting, the so-called Nelson rules [Nelson, 1984]. Nelson rules are a method of determining if some measured variable is consistent or unpredictable, and are known to the person skilled in the art. Additional quality control methods include the use of Hotelling's $T^2$ and squared prediction error (SPE) control charts. Figure 5 and Example 5 show an example of such procedure whereby an exhaled metabolite fulfils the Nelson rules. In the proposed method, the quality control test is preferably passed prior the breath test.

In a further aspect, the method of the present disclosure relates to an embodiment, wherein the chemical substance is a drug, a drug-related metabolite or a drug-regulated metabolite.

A chemical substance is herein defined as preferably an organic molecule. In certain preferred embodiments , the said organic molecule is not naturally present in a subject. The chemical substance is preferably a drug, a drug-related metabolite or a drug-regulated metabolite. The term drug herein refers preferably to a substance that if administered to a human or to a non-human animal, produces a biological effect. Biological effect is preferably herein defined as change to physiology and/or to psychology of a subject. Non limiting examples of such changes include change in blood pressure, pulse, body temperature, composition of the bodily fluids, composition of the blood, modulation of metabolism processes, modulation of activity of enzymes, modulation of activity of liver enzymes A pharmacological drug, also referred to herein as a medication or a medicine, is used preferably to treat, cure and/or prevent a specific disease that a subject, herein a human and/or a non-human animal, has been diagnosed with.

Drugs that are present in subjects undergo reactions catalyzed by enzymes present in the subject. This process is comprised within the broad definition of metabolism and may contribute to removal of unnecessary substances from the organism. The enzymes that take part in such processes are referred to as metabolic enzymes. It is possible that a drug present in a subject may be at least in part processed by the enzymes present in the subject and may be at least in part present in its reacted form - a product of an enzymatic reaction. In other words, it is possible that a drug will be present in its metabolized form. Such metabolized form of a drug is preferably referred to as a drug-related metabolite. It is possible than more than one drug-related metabolite is present, and more than one metabolic pathway leads to the formation of different drug-related metabolites. In the Example 3 of the present disclosure, determination of the concentration of a chemical substance according to the method of the present invention is discussed, wherein the previously trained mathematical model relies on peaks in the mass spectrum as predictors, that are due to drug-related metabolites.

As a result of the presence of a drug in a subject, the metabolic pathways of the subject may be altered. It is herein understood that the said metabolic pathways of the subject may be altered to an extent that is dependent of the concentration of a drug in the subject. A drug or a drug-related metabolite may for example inhibit a metabolic enzyme. A drug or a drug-related metabolite may also activate a metabolic enzyme. Finally, a drug or a drug related metabolite may modify the activity of a metabolic enzyme, changing the substrate scope and/or the chemical reaction catalysed by such an enzyme. Thus, due to the presence of a drug, the concentrations of certain metabolites may be altered. Such metabolites, the concentration of which is altered upon the presence of a drug, are referred to as a drug-regulated metabolite. It will be noted that in the method of the present invention, the concentration of a chemical substance in a subject can be determined based on observing a drug-regulated metabolite in the mass spectra collected for samples of breath exhaled by a subject. It will be further noted that a drug-regulated metabolite is preferably not derived from a drug.

According to the invention, a chemical substance is a drug-regulated metabolite. In the Example 6, the methods of the present invention is used to determine the levels of metabolites belonging to tyrosine metabolism pathway. The said metabolites of the tyrosine metabolism pathway include tyrosine, tyramine, dopamine and phenylalanine. Their level in a subject modulated due to the presence of a drug, or drugs in a subject, therein valproate, among others. Therefore, tyrosine, tyramine, dopamine and phenylalanine are examples of drug-regulated metabolites, herein antiepileptic drug-regulated metabolites. The level of a metabolite herein can be represented as concentration of the said metabolite in a subject or as abundance as measured in the mass spectrum, in other words intensity or nAUC parameter of a peak at m/z due to the said metabolite.

In a further aspect, the *ex vivo* method for determining the concentration of a chemical substance relate to an embodiment, wherein the concentration of the chemical substance in the subject is determined in real time.

As disclosed herein, the methods for determining the concentration of substance in a subject are typically time consuming and require preparatory work (for example separation of blood components). It is desirable to reduce the time

required for such measurements. Preferably, in the method of the present invention, the concentration of a chemical substance in a subject is determined in real time. The term "in real time" herein means that after provision of an exhaled breath sample to the mass spectrometer, the determination of the concentration of the chemical substance in a subject can be performed in less than 10 minutes, more preferably it can be performed in less than 5 minutes, most preferably in can be performed in less than 1 minute.

Figure 9 and Example 3 presents the correlation between the serum concentration of total chemical substance in a subject (herein: valproate, an antiepileptic medication) as measured by using state-of-the-art therapeutic drug monitoring approach and the determination using the method according to the present invention. Panel B presents correlation between measured and predicted free serum concentration of valproate. There is very significant correlation between the parameters measured by using the presently disclosed method, and those measured by using a more invasive approach, comprising blood analysis. It will be noted that the method of the present invention allows for determination of a concentration of a chemical substance in a subject in a real time, as opposed to the blood analysis-based methods. It is herein noted that exhaled VPA (and its metabolites) should mirror the free fraction of VPA (rather than the total VPA), as protein-bound VPA cannot be detected in breath and only the free fraction will undergo further metabolism.

In a further aspect, the present disclosure relates to a method for therapeutic drug monitoring, the method comprising: (a) providing a sample of breath exhaled by the subject to a mass spectrometer; (b) collecting the mass spectra of substances present in the exhaled breath in positive and/or negative mode; (c) analyzing the mass spectra using a previously trained mathematical model; and (d) determining the concentration of the chemical substance in the subject based on the analysis of the mass spectra of the exhaled breath; wherein the previously trained mathematical model relies on the signal intensity or area under the curve of selected m/z peaks in the mass spectra as predictors.

Herein, the chemical substance is preferably a drug or a drug-related metabolite, wherein the said drug is subject of therapeutic drug monitoring. According to the state of the art, therapeutic drug monitoring comprises the measurement of a concentration of a medication preferably in blood of a subject, preferably in the course of treatment of the subject with the medication. Knowledge of the drug concentrations in the subject allows adjusting the dose of drug required for the treatment. Several drugs show desired biological effect at a particular concentration, at the same time they show side effects at a different concentration. If the concentration at which the drug shows side effects is higher than the concentration at which the drug shows the desired activity, the drug can be used therapeutically, that means it can be administered to a subject in the need thereof. The range from the lowest concentration at which the desired effect can be observed to the lowest concentration at which the undesired side effects are observed is defined as a therapeutic window. If a drug is characterized by a narrow therapeutic window (that means the said range between the lowest concentration at which the desired effect can be observed to the lowest concentration at which the undesired side effects are observed is preferably less than 20 % of the lowest concentration at which the desired effect can be observed), it is desired to control the dose of the drug, and therapeutic drug monitoring is particularly useful in such cases.

By using the methods of the present invention, it is possible to determine the concentration of drugs and/or drug-related metabolites and/or endogenous metabolites in subjects, that are patients receiving medication and subjected to therapeutic drug monitoring of said medication. Thus, the methods of the present invention are useful in therapeutic drug monitoring. It is noted that the methods for therapeutic drug monitoring of the present invention are not invasive, as it is not necessary to obtain a sample of blood of a subject. Instead, therapeutic drug monitoring is performed by using samples of breath exhaled by a subject.

In other words, in a further aspect the method for determining the concentration of a chemical substance in a subject of the present invention relates to an embodiment, wherein the chemical substance is a medication, wherein the subject is treated with the medication, and wherein the determined concentration of the medication in the subject is used for therapeutic drug monitoring.

In the method of therapeutic drug monitoring of the present invention, a sample of breath exhaled by the subject is provided to a mass spectrometer using the methods as in the method of determining the concentration of a chemical substance in a subject of the present invention, as disclosed herein.

In the method of therapeutic drug monitoring of the present invention, the mass spectra of substances present in the exhaled breath are collected in negative and/or positive mode according to the method of determining the concentration of a chemical substance in a subject of the present invention, as disclosed herein.

In the method of therapeutic drug monitoring of the present invention, the mass spectra of substances present in the exhaled breath are analyzed using a previously trained mathematical model, according to the method of determining the concentration of a chemical substance in a subject of the present invention, as disclosed herein. In the said method of therapeutic drug monitoring, the previously trained mathematical model relies on the signal intensity or area under the curve of selected m/z peaks in the mass spectra as predictors.

In the method of therapeutic drug monitoring of the present invention, the concentration of the chemical substance in the subject is determined based on the analysis of mass spectra of exhaled breath, according to the method of determining the concentration of a chemical substance in a subject of the present invention, as disclosed herein.

Based on the evidence presented herein, we propose that the method for therapeutic drug monitoring of the present invention may serve as a companion diagnostic approach to minimize drug side effects and frustrating drug trial and error periods. Features that make it useful for the hospital in- and out-patients setting include non-invasiveness and real-time results. It is thus useful for chronically ill patients and children patients that need to be examined in real time during an outpatient consultation. Together with the method for determining concentration of a drug in a subject, allows proposing a clinical decision-making workflow based on the methods of the present invention (Figure 6).

Therapeutic drug monitoring can be applied to different drugs belonging to different drug classes. The non-limiting examples of monitored therapeutic drugs, that means drugs that are subjected to TDM, include anticonvulsants (phenytoin, carbamazepine, phenobarbital, primidone, valproate, clonazepam, gabapentin, lamotrigine), cardioactive drugs (digoxin, procainamide, *N*-acetylprocainamide, quinidine, lidocaine, amiodarone, flecainide, mexiletine, propranolol, verapamil, tocainide), anti-asthmatic drugs (theophylline, caffeine), antidepressants (amitriptyline + nortriptyline, nortriptyline, doxepin + nordoxepin, imipramine + desipramine, amoxapine, fluoxetine + norfluoxetine, sertraline, paroxetine), immunosuppressants (cyclosporine, tacrolimus, sirolimus, everolimus, mycophenolic acid), antineoplastic drugs (methotrexate, busulfan, 5-fluorouracil), and antibiotics (amikacin, gentamicin, tobramycin, vancomycin, ciprofloxacin, chloramphenicol, isoniazid, rifampin, ethambutol). The methods of therapeutic drug monitoring of present invention are not limited to any of these drugs, which are given as examples only.

In certain embodiments of the method of therapeutic drug monitoring of the present disclosure, medications can be administered as monotherapies. In this case, a subject is dosed with a single medication preferably according to the prescription by physician. Medication can also be prescribed in a form of a multi-therapy, wherein two or more medications are used to dose the subject during the same period of time, so that periods of treatment with different medications overlap. Therapeutic drug monitoring can be applied to selected medication both in the case when it is dosed alone, or if it is dosed in a combination with other medications.

Thus, in a further aspect, the method for therapeutic drug monitoring of the present disclosure relates to an embodiment, wherein therapeutic drug monitoring is performed for a subject treated under mono- or multi-therapy regime.

The method of the present invention can be used for therapeutic drug monitoring in a subject treated with one drug. Such situation is also referred to as a monotherapy regime. The method of the present invention can also be used for therapeutic drug monitoring for a subject treated under a multitherapy regime. A multitherapy regime refers to a situation when a subject is treated with more than one drug during the same period of time. The subject may herein be treated with two, three, four or more than four drugs at the same time. Drugs can be administered separately or as cocktails (i.e. in mixtures comprising more than one drug). As shown in Example 1 and Figure 7, it has been demonstrated in the monotherapy and multi-therapy of epilepsy that a chemical substance can be detected in a subject not only if it is the only substance that the subject is receiving, but also when subject is treated with one or two other drugs within the same period of time.

In a further preferred aspect, the method for therapeutic drug monitoring of the present disclosure relates to an embodiment, wherein therapeutic drug monitoring is used for determination of subject compliance, and/or response to the drug and/or occurrence of drug-related side effects in the subject.

Therapeutic drug monitoring according to the methods of the present invention is useful for determining subject compliance. Subject compliance is defined preferably as a degree to which a subject, preferably a patient undergoing therapy, follows medical advice. For example, the subject may be prescribed to take a medication 2 times a day over a month period. In case of lack of compliance, the subject would fail to take the medication on one or on several occasions. This would be reflected in the determined concentration of said drug in the subject, determined according to the methods of the present invention, which would appear lower in the periods corresponding to the lack of compliance.

Therapeutic drug monitoring according to the methods of the present invention allows accurate determination of a concentration of a drug in a subject at any selected time. Availability of time-dependence of the drug concentration in a subject allows studying its correlation with the clinical outcome of treatment. Clinical outcome may include alleviation of symptoms and/or occurrence of side effects. Therefore, therapeutic drug monitoring is useful in determining whether observed side effects are due to the treatment.

Drug pharmacodynamics (PD) is related to the effect of drugs. Usually, inter-individual variability in PD processes is greater than the inter-individual pharmacokinetic variability. As a result, failure to properly account for the variability in the PD of a drug may cause therapy failure or toxicity for individual patients.

In a further aspect, the methods of the present disclosure relate to an embodiment, wherein determined concentration of a chemical substance in a subject is used in the context of pharmacometabolomics. Pharmacometabolomics involves the measurement of metabolite levels following the administration of a drug or a medication. Therefore, in other words, in this embodiment of the methods of the present invention, wherein the chemical substance is a drug-regulated metabolite, that can also be referred to as medication-regulated metabolite. Accordingly, the methods of the present invention encompassed in this embodiment are useful in monitoring the effects of the drug (or medication) on certain metabolic pathways. This way, detailed mapping of drug effects on metabolism and the pathways that are implicated in mechanism of variation of response to treatment that can explain inter-individual differences in treatment

outcome can be provided.

Table 1 and Example 6 illustrate the metabolites, the level thereof in the subjects with epilepsy and treated with valproate, as measured according to the methods of the present invention, is upregulated or downregulated in different groups of subjects treated with anti-epileptic drugs (for example, with valproate). The level of a metabolite herein can be represented as concentration of the said metabolite in a subject or as abundance as measured in the mass spectrum, in other words intensity of nAUC parameter of a peak at m/z due to the said metabolite. By upregulated, it is understood that the level is increased, and by downregulated, it is understood that the level is decreased. The level of a metabolite can be expressed as relative level, compared to an average of a group of subjects, or as compared between the subjects. As disclosed herein, the levels of metabolites can be obtained from mass spectra of substances present in samples of exhaled breath. In subjects not responding properly to antiepileptic drugs (as defined by the number of seizures) the levels of tyrosine, tyramine, dopamine and phenylalanine, as measured according to the methods disclosed herein, were found to be downregulated. The association between downregulation of tyrosine metabolism and increased number of seizures (i.e. not responding adequately to medication) may be rationalized by the fact that neurotransmitter dopamine, which is known for its anti-epileptic action [Tripathi & Bozzi 2015], since it is synthesized from tyrosine and phenylalanine [Fernstrom 1990]. In subjects suffering from side effects (as defined by somnolence and irritability), the levels of amino acids including proline, glutamine, glutamic acid, lysine, aspartic acid, and asparagine, as well as further metabolites associated with the metabolic pathways of said metabolites, as measured by the methods of the present invention, are upregulated in comparison to healthy subjects. A strong association between upregulated amino acid metabolism and urea cycle and side effects *(i.e.* somnolence and acute irritability) is shown. Increased levels of proline and $\gamma$-aminobutyric acid were observed in subjects suffering from the side effects. Proline and $\gamma$-aminobutyric acid have been associated with uptake of valproate, an antiepileptic medication [Löscher 2002; Clelland et al. 2016].

According to the present invention, the concentration of a chemical substance in a subject is determined based on the analysis of the mass spectra of the exhaled breath; wherein the previously trained mathematical model relies on the signal intensity or area under the curve of selected m/z peaks in the mass spectra as predictors, wherein the predictors are the m/z peaks due to drug-regulated metabolites. As disclosed herein, it is possible to use any predictors, as long as their changes are associated with the changes in the concentration of a chemical substance in a subject. Table 1 and Example 6 show that the levels of proline, glutamine, glutamic acid, lysine, aspartic acid, asparagine, tyrosine, tyramine, dopamine and phenylalanine are different among different subjects suffering from epilepsy and being treated with drugs, e.g. with valproate and/or other antiepileptic drugs. These chemical substances can also be referred to as drug-regulated metabolites, herein antiepileptic drug-regulated metabolites.

The methods for therapeutic drug monitoring of the present invention can be useful in determining the estimate of side effect and/or probability estimate of drug response based on the above-mentioned drug-regulated metabolites. The collected mass spectra of substances present in the exhaled breath can also be used to generate mathematical models for determining the estimate of side effect and/or probability estimate of drug response based on the levels of different metabolites that can be observed in the mass spectra of breath exhaled by subjects. Table 1 and Example 6 show that the levels of proline, glutamine, glutamic acid, lysine, aspartic acid and asparagine are upregulated in patients suffering of side effects, whereas tyramine, tyrosine, phenylalanine and dopamine are downregulated in non-responders to pharmacotherapy. Thus, in another embodiment, the present invention relates to a method for therapeutic drug monitoring, the method comprising: (a) providing a sample of breath exhaled by the subject to a mass spectrometer; (b) collecting the mass spectra of substances present in the exhaled breath in positive and/or negative mode; (c) analyzing the mass spectra using a previously trained mathematical model; and (d) determining the risk estimate of side effects of a drug and/or probability estimate of drug response in the subject based on the analysis of the mass spectra of the exhaled breath; wherein the previously trained mathematical model relies on the signal intensity or area under the curve of selected m/z peaks in the mass spectra as predictors. Herein, a subject is preferably a patient treated with a drug, more preferably a subject is a patient that requires therapeutic drug monitoring. Herein, in the previously trained mathematical model, a target value comprises the risk estimate of side effects of a drug, and/or probability estimate of drug response. Further herein, the predictors are preferably m/z peaks due to drug-related and/or drug-regulated metabolites.

In the method of therapeutic drug monitoring of the present disclosure, wherein the risk estimates of side effects of a drug and/or probability estimate of drug response in the subject is determined, a sample of breath exhaled by the subject is provided to a mass spectrometer using the methods as in the method of determining the concentration of a chemical substance in a subject of the present invention, as disclosed herein.

In the method of therapeutic drug monitoring of the present invention, wherein the risk estimate of side effects of a drug and/or probability estimate of drug response in the subject is determined, the mass spectra of substances present in the exhaled breath are collected in negative and/or positive mode according to the method of determining the concentration of a chemical substance in a subject of the present invention, as disclosed herein.

In the method of therapeutic drug monitoring of the present invention, wherein the risk estimates of side effects of a drug and/or probability estimate of drug response in the subject is determined, the mass spectra of substances present in the exhaled breath are analyzed using a previously trained mathematical model, according to the method of determining

the concentration of a chemical substance in a subject of the present invention, as disclosed herein. In the said method of therapeutic drug monitoring, the previously trained mathematical model relies on the signal intensity or area under the curve of selected m/z peaks in the mass spectra as predictors.

Figure 8 shows the map of the probability of epileptic patients to suffer side effects and/or not respond to medication. The probability was computed using Random undersampling boosting (RUSBoost) algorithm. In this map, patients with high probability of having side effects and not responding to drugs will appear towards the top-right corner.

Random undersampling boosting (RUSBoost) is especially effective at classifying imbalanced data, meaning some class in the training data has many fewer members than another. RUS stands for Random Under Sampling. The algorithm takes N, the number of members in the class with the fewest members in the training data, as the basic unit for sampling. Classes with more members are under sampled by taking only N observations of every class. In other words, if there are K classes, then, for each weak learner in the ensemble, RUSBoost takes a subset of the data with N observations from each of the K classes. The boosting procedure follows the procedure in Adaptive Boosting for Multiclass Classification for reweighting and constructing the ensemble.

It will be noted that methods to calculate predicted probability score of epileptic patients to suffer side effects and/or not respond to medication are not limited to RusBOOST method. Other classification methods, including Support vector machines, Neural networks, Naive Bayes classifier, Decision trees, Discriminant analysis and Nearest neighbors are also useful for this purpose.

It will be further noted that according to the methods of the present invention it is possible to measure the level of metabolites that belong to different metabolic pathways. Therefore, by measuring the level of said metabolites and comparing them with each other, it is also possible to compare the activities of said different metabolic pathways. As demonstrated in the Example 3, it is possible to perform such an analysis by relying on the values of predictors determined in the course of the method of therapeutic drug monitoring of the present invention. Figure 3 presents the time dependence of the relative activity of two oxidation pathways of valproate, β-oxidation that leads to 3-heptanone, and ω1-oxidation that results in 2-propyl-4-pentanolactone. The activities of both oxidation pathways are presented herein as a ratio between the nAUC parameters for m/z peaks due to compounds belonging to each of the pathways, and presented in a logarithmic scale. Both increase of in 2-propyl-4-pentanolactone to 3-heptanone ratio, as well as decrease thereof can be observed over time, and observed ratios span more than 100-fold range. Herein, values of nAUC parameters for the m/z peaks due to both 3-heptanone and 2-propyl-4-pentanolactone (accordingly, peaks at m/z of 115.11176 and143.10659 in Figure 14) are predictors for the previously trained mathematical model of the present invention. Therefore, in a further aspect, the method for therapeutic drug monitoring of the present disclosure relates to an embodiment, wherein the contribution of different metabolic routes in metabolizing the therapeutic drug is calculated based on values of the predictors.

In a further aspect, the *ex vivo* method for determining the concentration of a chemical substance in a subject of the present disclosure, or the method for therapeutic drug monitoring of the present disclosure, relate to embodiments, wherein the chemical substance is a substance used as an anti-epileptic medication.

Epilepsy is a neurological disorder characterized by occurrence of episodes of intensive shaking, which may last from seconds to several minutes. Such episodes of intensive shaking characteristic to epilepsy are also referred to as epileptic seizures. Epileptic seizures have no immediate underlying cause. The cause of epilepsy remains unknown. However, some cases occur as a result of brain injury, stroke, brain tumor, brain infection and/or birth defects. In 70% of the cases, epileptic seizures can be controlled by using an anti-epileptic medication. Typically, antiepileptic medications are also known as anti-seizure medications, or anticonvulsants. Non-exhaustive list of the anti-epileptic medications includes phenytoin, carbamazepine, valproate, lamotrigine, levetiracetam, ethosuximide.

A further particular aspect of the methods of the present invention relates to an embodiment, wherein the antiepileptic medication is valproate.

Valproic acid is a chemical compound of a formula:

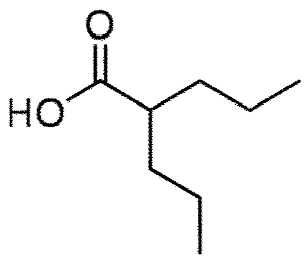

Valproic acid is used in the pharmaceutical formulations in acid form, as its sodium salts sodium valproate and valproate semisodium as medications to treat epilepsy, bipolar disorder, and to prevent migraine headaches. Common side effects include nausea, vomiting, sleepiness and dry mouth.

In a further particular aspect, the method of the present disclosure for determining the concentration of valproate

in a subject relates to an embodiment, wherein the total and free concentration of valproate is determined based on the peaks due to the ions $[C_7H_{15}O]^+$, $[C_6{}^{13}CH_{15}O]^+$, $[C_7H_{18}ON]^+$, $[C_7H_{13}O_2]^+$, $[C_6{}^{13}CH_{13}O_2]^+$, $[C_8H_{15}O_2]^+$, $[C_7{}^{13}CH_{15}O_2]^+$, $[C_8H_{15}O^{18}O]^+$, $[C_6{}^{13}C_2H_{15}O_2]^+$, $[C_8H_{18}O_2N]^+$, $[C_7{}^{13}CH_{18}O_2N]^+$, and/or $[C_7{}^{13}CH_{18}O_2]^-$ as predictors.

Figure 10 presents metabolic processes that lead to excretion of valproate and formation of valproate-related metabolites in a human subject. Less than three percent of valproate is directly excreted in urine, without any chemical modification. Most of valproate (80%) undergoes direct glucuronidation. Oxidation processes account for reminder of metabolic processes involving valproate. Oxidation at every carbon atom of valproate has been reported to occur in human subjects.

12% of valproate is modified with coenzyme A at the carboxylic group and shuttled to mitochondria to undergo processes that lead effectively to oxidation of β-position and formation of 3-oxovalproate. Valproyl coenzyme A is first oxidized to yield 2-ene-valproyl coenzyme A, which undergoes water addition yielding 3-hydroxyvalproyl coenzyme A. This product is then oxidised to 3-oxovalproyl coenzyme A, which in part is the substrate of the citric acid cycle, and in part is exported from mitochondria upon detachment of coenzyme A as 3-oxovalproate. 3-Oxovalproate undergoes spontaneous decarboxylation yielding 3-heptanone.

Valproate is oxidised in a cytochrome P450-dependent manner to 4-ene valproate, which can further be oxidized to 2,4-diene valproate. It should be noted that 2,4-diene derivative of valproate is also synthesized in humans starting from 2-ene-valproyl coenzyme A, which belongs to beta-oxidation pathway.

2.3 % of valproate are oxidised at position 5, yielding 5-hydroxyvalproate, which is further oxidised to 5-oxovalproate and 2-propylglutaric acid, also referred to as 2-PGA.

2.7% of valproate undergo oxidation at position 4, yielding 4-hydroxyvalproate. 4-hydroxyvalproate undergoes intramolecular esterifaction reaction, yielding 2-propyl-4-pentanolactone. It can however be further oxidised to 4-oxovalproate and 2-propylsuccinic acid, also referred to as 2-PSA. Finally, further oxidation of 4-oxovalproate at different sites may yield 2,3-heptanedione and 2,5-heptanedione.

3-Heptanone is a volatile compound of formula $C_7H_{14}O$, a product of valproate metabolism that gives raise to a number of charged ions upon electrospray ionization. In the process of ionization, a molecule forms adducts with positive ions like $H^+$ or $NH_4{}^+$, or loses charged moieties like $H^+$. In this process the formed ions include $[C_7H_{15}O]^+$, $[C_6{}^{13}CH_{15}O]^+$, $[C_7H_{18}ON]^+$. It is known to the person skilled in the art that as a natural content of $^{13}C$ isotope of carbon is 1%, it is expected that monoisotopic peaks in mass spectrum due to the species containing at least one $^{13}C$ atom appear. It is noted that the person skilled in the art will be aware that such substitution may occur at any carbon site in the molecule, and that differently substituted species will give raise to one peak corresponding to one particular chemical formula.

Different isomers of heptanedione, in particular 2,3-heptanedione and 2,5-heptanedione, are volatile compounds of formula $C_7H_{12}O_2$, and are products of valproate metabolism. In the process of ionization, a molecule forms adducts with positive ions like $H^+$ or $NH_4{}^+$, or loses charged moieties like $H^+$. The thus formed ions include $[C_7H_{13}O_2]^+$ and $[C_6{}^{13}CH_{13}O_2]^+$. It is known to the person skilled in the art that as a natural content of $^{13}C$ isotope of carbon is 1%, it is expected that monoisotopic peaks in mass spectrum due to the species containing at least one $^{13}C$ atom appear. It is noted that the person skilled in the art will be aware that such substitution may occur at any carbon site in the molecule, and that differently substituted species will give raise to one peak corresponding to one particular chemical formula.

2-propyl-4-pentanolactone is a volatile compound of formula $C_8H_{14}O_2$, a product of valproate metabolism that gives raise to a number of charged ions upon electrospray ionization. In the process of ionization, a molecule forms adducts with positive ions like $H^+$ or NH4+, or loses charged moieties like $H^+$. The formed ions include $[C_8H_{15}O_2]^+$, $[C_7{}^{13}CH_{15}O_2]^+$, $[C_8H_{15}O^{18}O]^+$, $[C_6{}^{13}C_2H_{15}O_2]^+$, $[C_8H_{18}O_2N]^+$, $[C_7{}^{13}CH_{18}O_2N]^-$, and $[C_7{}^{13}CH_{15}O_2]^-$. It is known to the person skilled in the art that as natural content of $^{13}C$ isotope of carbon is 1%, it is expected that monoisotopic peaks in mass spectrum due to the species containing at least one $^{13}C$ atom appear.

It is noted that the person skilled in the art will be aware that such substitution may occur at any carbon site in the molecule, and that differently substituted species will give raise to one peak corresponding to one particular chemical formula.

In certain embodiment of the present disclosure, the ions formed due to ionization of preferably 3-heptanone and/or 2-propyl-4-pentanolactone, the valproate-related metabolites, can be detected in breath exhaled by a subject using mass spectrometry and obtained mass spectrometry data can be used as predictors in a mathematical model to determine the concentration of valproate in a subject. However, the present invention is not limited to this embodiment, and different combinations of valproate-related metabolites that can be detected in the mass spectrometry analysis of exhaled breath and used to determine whether a subject is a user of valproate, remains within the scope of the present invention. Furthermore, the present invention is not limited to detecting valproate in a subject, it is applicable to any chemical substance. The present invention provides means for detecting any chemical substance in a subject, based on analysis of samples of breath exhaled by the subject by the mass spectrometry. The predictors as well as the mathematical model will vary depending on a chemical substance.

Figure 14 presents typical mass spectra of exhaled breath in both positive and negative mode, compared both for subjects using valproate and those who are not valproate users. In the breath exhaled by a valproate user, the peaks due to

3-heptanone adducts at m/z = 115.1118 corresponding to $[C_7H_{14}O + H]^+$ and at m/z = 132.1383 corresponding to $[C_7H_{14}O + NH_4]^+$ are observed in the positive mode. These peaks are not observed in the mass spectrum of breath exhaled by a subject that is not a valproate user. Moreover, in the breath exhaled by a valproate user, the peak due to different isomers of heptanedione at m/z = 129.0910 corresponding to an adduct $[C_7H_{12}O_2 + H]^+$ is observed. In the mass spectra of breath exhaled by subjects that are not valproate users, this peak has significantly reduced intensity and area under the curve parameter. Furthermore, in the breath exhaled by a valproate user, the peaks due to 2-propyl-4-pentanolactone-derived ions at m/z = 143.1066 corresponding to $[C_8H_{14}O_2 + H]^+$ and at m/z = 161.1365 corresponding to $[C_8H_{14}O_2 + NH_4]^+$ are observed in a positive mode, while the peak at m/z = 144.1111 corresponding to $[C_7{}^{13}CH_{14}O_2 - H]^-$ is observed in a negative mode. These peaks are either absent or significantly reduced across the mass spectra of breath exhaled by subjects that are not users of valproate.

It is noted that determination of the concentration of valproate in a subject based on analysis of the collected mass spectra of the samples of breath exhaled by a subject, wherein only the m/z peaks due to valproate is considered would be prone to error, as subjects not treated with valproate exhale other endogenous compounds (e.g. octanoic acid) that are isomers of valproic acid (same exact mass and also giving raise to a peak of m/z of 144.11106 in mass spectra collected in a negative collection mode - see Figure 14), and hence cannot be resolved by SESI-HRMS. Similar situation is seen for heptanedione (different isomers), a metabolite of valproate, as its isomers are also present in the breath exhaled by subjects, which are not receiving valproate (Figure 14).

Subjects may differ in the activities of different metabolic pathways that a chemical substance is subjected to. As a result, the concentrations of different chemical-substance-related metabolites may be different in different subjects. Furthermore, the said concentrations may change with time. It will be noted that the values of different predictors may differ between the subjects. Furthermore, it will also be noted that the values of different predictors may change for one particular subject over time.

Figure 3 presents the time dependence of the relative activity of two oxidation pathways of valproate, $\beta$-oxidation that leads to 3-heptanone, and $\omega$1-oxidation that results in 2-propyl-4-pentanolactone. The activities of both oxidation pathways are presented herein as a ratio between the nAUC parameters for species due to each of the pathways, and presented in a logarithmic scale. Both increase of in 2-propyl-4-pentanolactone to 3-heptanone ratio, as well as decrease thereof can be observed over time, and observed ratios can be smaller than 0.5, they can also be higher than 32.

In a further particular aspect, the methods of the present disclosure relate to an embodiment, wherein the chemical substance is a substance used as an anti-cancer medication. In a preferred embodiment, the anticancer medication is methotrexate.

Figure 11 and Example 7 provide further evidence on the feasibility of non-invasive monitoring of drugs via exhaled breath analysis. It shows the time profiles of methotrexate (MTX) serum concentration and signal intensity of a compound detected in the breath exhaled by the same leukemia patient during two separate sessions of treatment with MTX. In this case, 1 mL of serum was required to estimate MTX concentrations via an immunoassay. A clear correlation between the signal intensity of an ion detected in breath at m/z 373.064 and the MTX serum concentrations is observed. It is conceivable that based on the methods disclosed herein, it is possible to determine the concentration of methotrexate in a subject according to the methods of the present invention, wherein the previously trained mathematical model relies on predictors comprising said peak at m/z 373.064. It is further conceivable that therapeutic drug monitoring of methotrexate can be performed according to the methods of the present invention. It will be noted that the identity of a metabolite that gave raise to the said peak at m/z of 373.064 is not known. Therefore, it is further exemplified that the methods of the present invention do not require the knowledge of the molecular basis of metabolites that give raise to m/z peaks, the intensity or the nAUC of which are used as predictors. It is only required that said predictors depend on the concentration of the chemical substance in subjects.

In another aspect, the present disclosure relates to an apparatus for use in the methods of the present invention, the apparatus comprising (a) a disposable mouthpiece for collecting a sample of breath exhaled by the subject; (b) a connector for delivering the collected sample to the ionization device; (c) a mass spectrometer comprising an electrospray ionization module and a detection module; (d) a computer interface for analysis and determination of the concentration of the substance in the subject; wherein the apparatus is configured for carrying out a previously trained mathematical model used in the determination of the concentration of the chemical substance in the subject, wherein the mathematical model relies on the signal intensity or area under the curve of selected m/z peaks in the mass spectra as predictors, and wherein the mass spectrometer is calibrated for analyzing breath samples.

Figure 1 illustrates the exemplary embodiment of the apparatus of the present disclosure. Herein, a mouthpiece is defined as a hollow object with opening of both sides. One side of the mouthpiece is preferably configured to be attached to a mouth of a subject and/or allow for a tight flow of exhaled breath into the mouthpiece. The other side of the mouthpiece is configured to preferably be attached to a connector, so that the flow of exhaled breath from the mouth of the subject preferably through the mouthpiece can be directed into the connector. Optionally, within the opening of the mouthpiece a filter may be placed and/or configured in such a way, that the breath exhaled through the mouthpiece flows through the filter. Any mouthpiece that fulfils these requirements and/or is suitable for directing a sample of breath exhaled by a subject

can be used in the apparatus of the present invention. For example, any mouthpiece known to a person skilled in the art to be suitable as mouthpiece for spirometric measurement, can be used in the apparatus of the present invention. Preferably, mouthpieces with filters such as M.R.D Filter MRSF-22 (Medicalrd, www.medicalrd.com), MADA Spirometry filter 83 (AMC AG, www.amc-ag.ch), Microguard IIC (Vyaire, www.vyaire.com) or Aerovent-Geräteschutzfilter Spirometer (HUM, www.hum-online.de) are being used in the apparatus of the present invention in order to prevent transmission of viruses and bacteria.

A non limiting example of a mass spectrometer suitable for application in the apparatus of the present disclosure is the Orbitrap HRMS. A further non limiting examples of a mass spectrometer within the Orbitrap HRMS family suitable for such application is the Q Exactive Plus Mass Spectrometer.

In a particular aspect, the apparatus for use in the methods of the present disclosure relates to an embodiment, wherein the connector is configured for optimal delivery of compounds that give raise to peaks with m/z in the range of 50-1000.

It is known to a person skilled in the art that any gas transfer may suffer loses because of absorption at walls of the connector and/or because the connector is not completely sealed. Depending on the material of the connector, and/or the flowrate of gas and/or the dimensions of the connector, in particular its cross-section and length, different type of molecules will be optimally delivered. Herein, optimization is performed either by monitoring TIC and maximizing its value, or by monitoring signals due to substance(s) comprised in the standard gas mixture and maximizing their observed intensities. In the apparatus of the present invention, the connector is configured for optimal delivery of volatilome. Therefore, the said connector is configured for optimal delivery of compounds that give raise to m/z in the range from 50 to 1000. Preferably, the said connector is configured for optimal delivery of compounds that give raise to peaks in a mass spectrum with an m/z ratio in the range from 50 to 200. In a particular embodiment of the present invention, the optimal delivery of compounds that give raise to peaks with m/z in the range of 50-1000 is achieved by using a sampling tube or a connector that comprises tubing made of coated stainless steel with inner diameter of 3 mm, outer diameter of 6 mm, and length of 50 cm.

In a more specific aspect, the apparatus for use in the methods of the present disclosure relates to an embodiment, wherein the apparatus is configured to perform the analysis in real time.

In the method of the present disclosure provision of a breath sample expired by a subject, collecting the mass spectra thereof, and analysis the said spectra by using a previously trained mathematical model, are performed together. In line with that, an apparatus of the present invention integrates means for provision of a sample of exhaled breath, mass spectrometer, preferably configured for measurements of samples of exhaled breath, and computer interface configured to analyze the data. Therefore, the apparatus of the present invention is configured to complete the analysis preferably while the subject is still providing samples of exhaled breath to the apparatus, without the need for any post-processing or human intervention. The term "in real time" in the current context means that after provision of an exhaled breath sample to the mass spectrometer, the determination of the concentration of a chemical substance in a subject can be performed in less than 10 minutes, more preferably it can be performed in less than 5 minutes, most preferably in can be performed in less than 1 minute.

In a further aspect, the apparatus for use in the methods of the present disclosure relates to an embodiment, wherein the computer interface is configured to perform a quality control of the sample analysis based on the variability between repeated measurements and measurements with standardized gas composition, preferably containing compounds that give raise to m/z in the range of 100-200.

The standard gas mixture comprises substances that behave similar in the mass spectrometry setup for breath sample analysis as the samples of exhaled breath. That means, the standard gas mixture preferably contains substances that give raise to m/z peaks, which are in the same range as the peaks observed due to volatilome. Preferably, components of the standard gas would give raise to peaks of m/z of in the range from 50 to 200. The non-limiting examples of substances that are useful as components of the standard gas preferably include acetone, isoprene, 2-butanone, 2-pentanone, toluene, styrene, mesitylene, and terpene. These substances give raise to peaks of m/z corresponding to 59.0491414, 69.0698769, 73.0647915, 87.0804415, 93.0698769, 105.0698769, 121.101177, and 137.1324771, respectively.

In another aspect, the present disclosure relates to use of the apparatus disclosed herein in therapeutic drug monitoring of subjects undergoing disease treatment.

In a further aspect, the use of the apparatus of the present disclosure in therapeutic drug monitoring of subjects undergoing disease treatment relates to an embodiment, wherein the disease is epilepsy or cancer.

In the further aspect, the present disclosure relates to an off-line sample collection tube, comprising: (a) a disposable mouthpiece; (b) a container configured for holding a gas sample; (c) a sealed connection valve, wherein the sample is collected by exhaling into the mouthpiece, wherein the container can be sealed upon sample collection, and wherein the sealed connection valve allows for connecting the sample collection tube to the apparatus of the present invention.

Any mouthpiece that is suitable for use with the apparatus of the present disclosure is suitable for use with an off-line sample collection tube of the present disclosure. For example, any mouthpiece known to a person skilled in the art to be

suitable as mouthpiece for a spirometric measurement, can be used in the apparatus of the present invention. Preferably, MADA filters 83 Orange are used in the apparatus of the present invention.

A container configured to holding a gas sample can be made of any material that is chemically inert, and/or that does not absorb gases, and/or that is not permeable to gases. The container configured to holding a gas sample can preferably be made of glass. Breath samples, also referred to as samples of breath exhaled by subjects, are collected into gas containers configured for collection and storage of breath exhaled by a subject, preferably sealed and/or attached to the mass spectrometer, preferably configured for provision of gas samples, preferably exhaled breath samples.

The container comprises at least one sealed connection valve. A gas sample, preferably a sample of a breath exhaled by a subject, can be introduced to the container through a sealed connection valve, preferably by using a disposable mouthpiece. The same connection valve can be used to seal the gas container for storage and/or for transport, and/or to attach the gas container to a mass spectrometer, preferably configured for mass spectrometry measurements of gas samples, preferably of samples of exhaled breath. Preferably, more than one sealed connection valve may be present in the gas container of the present invention. Preferably, the current invention may contain two sealed connection valves, preferably on the opposite sides of the container. One sealed connection valve may preferably be configured for introducing a sample of breath exhaled by a subject into the container, and preferably another one configured for attaching the gas container to a mass spectrometer, preferably configured for mass spectrometry measurements of gas samples, preferably samples of exhaled breath.

In another aspect of the present disclosure, as illustrated by Example 8, preferred gas collection containers are bags, preferably Nalophan bags or Teflon bags. These bags are made of poly(ethylene terephtalate) and are useful for collecting and storing volatile compounds at low concentration, for example less 10 $\mu g/m^3$. The breath sample is subsequently transported to the mass spectrometer and delivered into the ion source, producing as a result a similar TIC as the one showed in Figure 4.

**References**

Beghi, E. et al., 2019. Global, regional, and national burden of epilepsy, 1990-2016: a systematic analysis for the Global Burden of Disease Study 2016. The Lancet Neurology, 18(4), pp.357-375.

Bregy, L. et al., 2014. Real-time breath analysis with active capillary plasma ionization-ambient mass spectrometry. J. Breath Res. 8 (2).

Clelland, C.L. et al., 2016. Evidence that COMT genotype and proline interact on negative-symptom outcomes in schizophrenia and bipolar disorder. Translational psychiatry, 6(9), p.e891.

Dasgupta, A., 2012/ Chapter 1 - Introduction to Therapeutic Drug Monitoring: Frequently and Less Frequently Monitored Drugs. In Ther. Drug Monit. pp 1-29.

Duran, J. A.; Abadin, J. A.; Sanchez, A.; Serrano, J. S., Free Plasma Fraction of Valproic Acid in Epileptic Patients. Drug Invest 1993, 5 (2), 108-113.

Fernstrom, J.D., 1990. Aromatic amino acids and monoamine synthesis in the central nervous system: influence of the diet. The Journal of Nutritional Biochemistry, 1(10), pp.508-517.

Kang, J. & Lee, M., 2009. Overview of Therapeutic Drug Monitoring. , pp.1-10.

Löscher, W., 2002. Basic pharmacology of valproate: A review after 35 years of clinical use for the treatment of epilepsy. CNS Drugs, 16(10), pp.669-694.

Nelson, L.S., 1984, "The Shewhart Control Chart-Tests for Special Causes". Journal of Quality Technology 16, no. 4 (October 1984), 238-239.

Schiller, Y., 2009. Seizure relapse and development of drug resistance following long-term seizure remission. Archives of Neurology, 66(10), pp.1233-1239.

Singh, K.D. et al., 2019. Standardization procedures for real-time breath analysis by secondary electrospray ionization high-resolution mass spectrometry. Analytical and Bioanalytical Chemistry.

Singh, K.D. et al., 2018. Translating secondary electrospray ionization-high-resolution mass spectrometry to the clinical environment. Journal of breath research, 12(2), p.027113. Available at: http://www.ncbi.nlm.nih.gov/pubmed/29411710.

Tripathi, P.P. & Bozzi, Y., 2015. The role of dopaminergic and serotonergic systems in neurodevelopmental disorders: A focus on epilepsy and seizure susceptibility. BioImpacts, 5(2), pp.97-102. Available at: http://dx.doi.org/10.15171/bi.2015.07.

Weaving G, Batstone GF, Jones RG. Age and sex variation in serum albumin concentration: an observational study. Ann Clin Biochem. 2016;53(1):106-111. doi:10.1177/0004563215593561.

**Brief description of Tables and Figures.**

Table 1 summarizes data on drug-regulated metabolites, the-levels of which are altered in subjects using antiepileptic drugs. Said levels can be used in determining the risk estimate of side effects of a drug and/or probability estimate of drug response in the subject based on the analysis of the mass spectra of the exhaled breath ($\log_2$ FC = log2(Fold Change); Adducts = different ions bound to the neutral species during the ionization process, compounds in the significant set as indicated in the analysis with MetaboAnalystR are denoted by $\surd$; in Average log2 FC column, values where m/z have different fold change direction are by ⫲; in m/z polarity column: Pos = Positive and Neg = Negative; in Adduct column, A = $[M + H]^+$, B = $[M + 1 + H]^+$, C = $[M - NH3 + H]^+$, D = $[M + NH4]^+$, E = $[M + H2O + H]^+$, F = $[M - H]^-$, and G = $[M + 1 - H]^-$)

Figure 1 illustrates a typical mass spectrometer configured for analysis of breath samples (samples of breath exhaled by a subject). In other words, a schematic of the real-time SESI-HRMS breath analysis platform is shown.

Figure 2 presents an analysis pipeline that includes constructing a response vector matrix, splitting the dataset into training and test set, applying feature reduction analysis to test set in order to identify predictors, training mathematical model based on selected predictors, applying selected predictors and mathematical model to the test set to determine the concentration of valproate in a subject, which matches well the serum concentration determined using the blood analysis, as determined in an immunoassay (total valproate concentration) and by GC-MS (free valproate concentration).

Figure 3 shows the time profile of $\beta/\omega_1$ oxidation of valproate assessed based on ratio of 2-propyl-4-pentanolactone and 3-heptanone for 11 subjects with multiple visits (i.e. that participated in the measurement of breath samples by mass spectrometry according to the methods of the present invention multiple times).

Figure 4 shows the effect the total ion current (TIC) detected by the mass spectrometer during a breath test.

Figure 5 shows a screenshot of the quality control app used to ensure high quality breath metabolomics data.

Figure 6 depicts the taught method to stratify patients and guide their therapeutic regime by breath analysis.

Figure 7 presents the cohort of patients that took part in the study of Example 1. The patients suffered from epilepsies of different origins, as indicated in the row epilepsy group. Group 1 consisted of patients with epilepsies of structural origin, group 2 consisted of patients suffering from genetic generalized and self-limited focal epilepsies, as well as epilepsies of unknown origin, and group 3 consisted of developmental and epileptic encephalopathies (DEE).
The range of pharmacotherapies is described in AED rows. The patients were receiving either a single drug or combinations of up to three drugs. The bottom three rows identify the clinical outcome as described by side effects (mild or severe), drug response (responder or non-responder), and EEG (normal or abnormal). The grey bars on the top show the concentrations (mg/L) of free and total serum VPA (level 0 was assigned to all the measurements of subjects' not taking VPA, missing bars in free VPA represents the unavailability of data). Dark (light) grey bars indicate that the measurements were outside (inside) the therapeutic range (i.e. 50-100 mg/L for total and 5-10 mg/L for free VPA). Thus, for example, patient 20 suffers from DEE. During the first visit (i.e. 20-1), the patient was receiving a combination of LTG, LEV, and VPA. Total serum VPA concentration was 104 mg/L (i.e. beyond the therapeutic range) and free serum VPA could not be measured. At this point in time, the patient had only mild side effects, but was not responding to the medication, and EEG was abnormal. On the second visit, the patient was unable to perform breath test. During the third visit (i.e. 20-3), LEV had been removed, total serum VPA concentration was 108 mg/L and free serum VPA concentration was 12.7 mg/L (both levels above the therapeutic range). Side effects were still mild, EEG

was back to normal, but the patient was still not responding to the pharmacotherapy. AED = antiepileptic drugs, EEG = electroencephalography, VPA = valproic acid, LTG = lamotrigine, and LEV = levetiracetam.

Figure 8 shows the computed risk estimates, based on altered metabolites described in Table 1 for drug therapy effects, as well as side effects for a cohort of patients receiving antileptic drugs.

Figure 9 shows the plots of systemic total (A) and free (B) VPA serum concentration determined (predicted) based on real-time breath mass spectra determined according to the method of the present invention vs. measured directly in serum.

Figure 10 shows different metabolic pathways that lead to clearing of valproate from a subject organism.

Figure 11 shows the time profiles of methotrexate serum concentration and signal intensity of a breath compound for the same patient.

Figure 12 further presents study participants as described in Example 1 and Figure 7. **(A)** Distribution of age among study participant. Tukey style boxplot for subject age grouped by gender for measurements with valproate and without valproate, boxes are overlaid with actual data points, labelled as subject number-visit number. **(B)** Shows the non-linear relationship between total and free valproate, solid line shows the quadratic fit line along with 95% confidence as dashed lines. **(C)** For subjects of this study, fraction of free valproate shows an inverse relationship with the age of participants. **(D)** Data shows another widely-known trend that for a given doses of total valproate under multi-therapy are lower than those of mono-therapy (denoted by cross).

Figure 13 shows the most important ions in a complex breath mass spectrum, as determined by an algorithm, to predict systemic VPA concentrations.

Figure 14 shows the most important predictors for determining the concentration of valproate in a subject according to the method of the present invention. The predictors herein are peaks in the mass spectra of samples of breath exhaled by subjects. Spectra collected in both positive and negative modes are shown. Comparison between users and non-users of valproate is shown.

Figure 15 shows the prediction of total (A and C) and free (B and D) VPA serum concentration of independent test-set using single m/z peaks as predictors.

Figure 16 shows the TIC during the deflation of a Nalophan bag containing a human breath sample (top panel). The bottom panel shows the corresponding average mass spectrum at the plateau of the TIC signal.

Figure 17(A-C) show the volcano plots after two sample t-test between training-set measurements of side effects vs no side effects (A), non-responders vs responders (B), and abnormal vs normal EEG (C). Each data point (N = 1005) is an ion measured in SESI-HRMS. Horizontal solid grey line represents the no change and vertical dashed grey line represents P-value cut-off of 0.015 in panel A and 0.05 in panel B. Different (data specific) P-value cut-offs were used to make sure ~10% of total ions appears in significant list, to allow proper downstream analysis via MetaboAnalystR. Points in black and medium grey represent ions corresponding to compounds from significantly enriched pathways, where black points are significant and medium grey points are not, actual numbers of these and remaining points are denoted by n at the bottom right side of each panel. Figure 17(D-F) show distribution of BH-adjusted P-values of two sample t-test for side effects (D), drug response (E), and EEG (F) based classification of measurements.

Figure 18 shows Real-time breath analysis offers a non-invasive window into altered metabolic pathways in patients not responding to pharmacotherapy and/or suffering of side effects. (A) SESI-HRMS breath analysis detected alteration in levels of several amino acids and associated compounds in epileptic patients. Figure shows a simple (unweighted, undirected, no loops or multiple edges) graph of amino acid metabolism (based on KEGG map01230: biosynthesis of amino acids). Each node is a compound, where node fill colour represents the mean $\log_2$ scaled fold change in side effects (yes vs no), and drug response (non-responder vs responders) dataset from training-set. Node border colour represents whether the compound was assigned to significant or background list via MetaboAnalystR. Only compounds from the significantly enriched pathways are coloured in different shades of grey. The rest are shown as white. Node with two colour (node split) denotes that compound was present under significantly enriched pathway of both datasets. (B-C) Density plot for predicted score and classes of having side effects (B) and drug response (C). Density curves are accompanied with actual data points, where each point represents one measurement from UKBB

dataset, shown in shades of grey based on clinically observed side effects (B) and clinically observed drug response (C). On predicted scores, a cut-off was assigned (based on Youden's index calculated using only training-set data) to separate predicted classes.

Various modifications and variations of the invention will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in the relevant fields are intended to be covered by the present invention.

The following examples are merely illustrative of the present invention and should not be construed to limit the scope of the invention which is defined by the appended claims in any way.

## EXAMPLES

### Example 1

59 subjects (32 males and 27 females) between the ages of 4 and 20 (see Figure 12A for details), undergoing treatment with antiepileptic drugs (AEDs) with the necessity of therapeutic drug monitoring (TDM) were enrolled in the study. Immediately after blood drawn for TDM, subjects performed prolonged exhalations directly into SESI-HRMS system, comprising SUPER-SESI ionization device (Fossil Ion Technology, Spain) coupled to a Q Exactive Plus HRMS (Thermo Fisher Scientific, Germany). During the course of study, wherever blood-based TDM were performed, subjects also provided exhalations, leading to multiple measurements from some subjects. In total 75 successful measurements (from 48 subjects) were performed. Study participants are good representation of global pediatrics epileptic population and include individuals under both mono- and multi-therapy, people switching between mono-and multi-therapy during different visits (e.g. subject number 16 and 34, Figure 7), and individuals with significantly different doses of same drug during different visits (e.g. subject number 41, Figure 7). In order to determine the feasibility of TDM of an example drug valproate by using mass spectroscopy of samples of breath exhaled by subjects, the subjects were divided into two groups i.e. valproate users and non-valproate users. Figure 7, also shows the serum concentrations of total and free valproate (non-protein-bound) in subjects, indicating the inter-and intra-subject variability. Free valproate is a clinically active form of valproate and its serum concentrations of free and total valproate exhibits a non-linear relationship, as observed for study participants (Figure 12B). Additionally, an inversely proportional relationship between participants' age and serum free valproate fraction was observed (Figure 12C), which might be explained by the fact smaller children have less serum albumin as compared to late teenagers (Weaving, 2016). Moreover, a different relationship between total valproate serum concentration and valproate dose for subjects with mono and multi-therapy compared to that known in the literature (Duran, 1993) was observed (Figure 12D).

### Example 2

For the subjects of Example 1 that were valproate users and participated in the analysis of samples of exhaled breath using mass spectrometry multiple times, the composition of the volatilome, and in particular concentrations of metabolites due to two different oxidation pathways, were followed over time. Figure 3 presents the time dependence of the relative activity of two oxidation pathways of valproate, $\beta$-oxidation that leads to 3-heptanone, and $\omega_1$-oxidation that results in 2-propyl-4-pentanolactone. The activities of both oxidation pathways are presented herein as a ratio between the nAUC parameters for m/z peaks due to compounds belonging to each of the pathways, and presented in a logarithmic scale. Both increase of in 2-propyl-4-pentanolactone to 3-heptanone ratio, as well as decrease thereof can be observed over time, and observed ratios span more than 100-fold range. Subjects 03 and 20 appear to have significant variation as compared to other subjects.

### Example 3

For the subjects of Example 1 that were users of valproate, real time analysis of exhaled breath by mass spectrometry according to the present invention has been performed to determine the concentration of valproate in the subjects. Independently, a blood-based therapeutic drug monitoring of valproate has been performed for comparison. The obtained data is shown in Figure 9. It has been shown that real time analysis of exhaled breath by mass spectrometry can predict serum concentration of free valproate with root mean square error (RMSE) were 12.4 and 1.7 mg/L for total and free VPA, respectively.

Reference Example 3

Figure 15 shows the prediction of total (A and C) and free (B and D) VPA serum concentration of independent test-set using matern 5/2 GPR method. Top row (A and B) corresponds to prediction result based on only peak at m/z equal to 143.1066 due to 2-propyl-4-pentanolactone as predictor, whereas bottom row (C and D) is based on peak at m/z equal to 115.1118 due to 3-heptanone as predictor. Patients not receiving VPA are shown by open squares, whereas patient on mono- or multi- VPA therapy are shown by open circles. Vertical grey box shows the reference therapeutic range of 50-100 mg/L for total VPA and 5-10 mg/L for free VPA. Solid grey line represents the identity (y = x) line.

Example 4

Figure 13 shows an example of a feature selection process. The set of patients as described in Example 1 was used. In this case, the training set consisted of 46 measurements of patients receiving antiepileptic drug valproic acid (VPA), for whom the serum concentration of total VPA concentration (in mg/L) was the target variable. Feature importance was computed using ReliefF and random forest algorithms. Predictor importance from both methods were combined to generate an overall normalized weight assigned to each predictor from positive (A) and negative (B) modes. Predictors with weights above an empirical cut-off of 0.1 as calculated by ReliefF algorithm (shown by dashed grey line) were selected for total VPA concentration prediction. Figure 14 shows the breath mass spectra at the selected important features for patients receiving VPA (black peaks) and patients receiving other antiepileptic pharmacotherapy (grey peaks). As expected, even by simple visual inspection of the breath mass spectra, one can appreciate that the signal intensity of these 12 mass spectral peaks was overwhelmingly more abundant in the patients taking VPA than in the patients taking other AEDs. These ions could be assigned to $[C_7H_{15}O]^+$, $[C_6{}^{13}CH_{15}O]^+$, $[C_7H_{18}ON]^+$, $[C_7H_{13}O_2]^+$, $[C_6{}^{13}CH_{13}O_2]^+$, $[C_8H_{15}O_2]^+$, $[C_7{}^{13}CH_{15}O_2]^+$, $[C_8H_{15}O^{18}O]^+$, $[C_6{}^{13}C_2H_{15}O_2]^+$, $[C_8H_{18}O_2N]^+$, $[C_7{}^{13}CH_{18}O_2N]^+$ and $[C_7{}^{13}CH_{15}O_2]^-$. The 12 features were associated to four unique molecules: VPA itself and three metabolites. Namely, i) 3-heptanone, which is a non-enzymatic end-product of the β-oxidation pathway of VPA; ii) 4-OH-γ-lactone, which is an end-product downstream the ω1-oxidation VPA pathway; and iii) a third metabolite with molecular formula $C_7H_{12}O_2$ thought to be heptanedione. This metabolite would also be produced downstream the ω1-oxidation route. The thus-trained regression method GPR can be applied to predictors extracted from new breath mass spectra (not previously trained) to predict the systemic total or free VPA fraction. Figure 9 shows the prediction of systemic drug concentration based on real-time breath mass spectra. Prediction of total (A) and free (B) VPA serum concentration of independent test sets. Patients not receiving VPA are shown by open squares, whereas patient on mono- or multi- VPA therapy are shown by open circles along with labels (patient ID-visit number). Vertical grey box shows the reference therapeutic range of 50-100 mg/L for total VPA and 5-10 mg/L for free VPA. Solid grey line represents the identity line (y = x). Root mean square error (RMSE) were 12.4 and 1.7 mg/L for total and free VPA, respectively. It has also been shown that free VPA is physiologically active and clinically relevant, which stresses the importance of measuring free VPA concentration. In spite of this, current clinical practice relies most often on total VPA blood concentrations, perhaps due to the fact that determination of free VPA requires large blood volumes (~ 5 mL), lengthy, and laborious mass spectrometric analyses requiring hours-to-days of laboratory work. In contrast, here we show the possibility of estimating free VPA concentrations in 15 minutes by the method taught herein. Moreover, the prediction is based in VPA and its metabolites, hence further insights could be gained on how the drug is metabolized. For example, 3-heptanone and 2-propyl-4-pentanolactone are drug metabolites from two different metabolic routes *(i.e.* β-mitochondrial vs $\omega_1$ pathways).

Example 5

A standard gas mixture is used for calibration of the apparatus of the present invention, SESI-HRMS, comprising SUPER-SESI ionization device (Fossil Ion Technology, Spain) coupled to a Q Exactive Plus HRMS (Thermo Fisher Scientific, Germany). The standard gas includes acetone, isoprene, 2-butanone, 2-pentanone, toluene, styrene, mesitylene, and terpene. These substances give raise to peaks of m/z corresponding to 59.0491414, 69.0698769, 73.0647915, 87.0804415, 93.0698769, 105.0698769, 121.101177, and 137.1324771, respectively. Figure 5 shows a screenshot of the control chart of the quality control app used to ensure high quality breath metabolomics data. Therein, a number of counts for a peak at m/z of 87.0804415, corresponding to 2-pentanone is plotted against time. The time series of the signal intensity fulfil the Nelson rules (Nelson, 1984), so the quality test is passed.

Example 6

The mass spectra of substances present in samples of exhaled breath collected for the group of patients as in Examples 1-4 were analysed to account for changes in relative levels of metabolites in subjects. As shown in Table 1, it has been found that in the group of subjects the levels of metabolites related to tyrosine metabolism, including tyrosine,

tyramine, dopamine, phenylalanine were significantly downregulated relative to the average levels of said metabolites in the group of subjects of the present study.

Example 7

Figure 11 shows the time profiles of methotrexate (MTX) serum concentration and signal intensity of a compound detected in the breath exhaled by the same leukemia patient during two separate sessions of treatment with MTX. In this case, 1 mL of serum was required to estimate MTX concentrations via an immunoassay. A clear correlation between the signal intensity of an ion detected in breath at m/z 373.064 and the MTX serum concentrations is observed.

Example 8

Figure 16 shows the TIC during the deflation of a Nalophan bag containing a human breath sample. The bottom panel shows the corresponding average mass spectrum at the plateau of the TIC signal. Most features detected in real-time are preserved by this off-line collection method.

Example 9

Endogenous metabolites altered in the training-set measurements of patients suffering from side-effects, or not-responding to pharmacotherapy i.e. non-responders, or those showing abnormal EEGs on the day of consultation were identified. The exhaled breath metabolites tend to be i) upregulated in children suffering from side effects as compared to no side effects and ii) downregulated in non-responders as compared to responders (Fig. 17). In contrast, abnormal EEG showed no significant change in the levels of exhaled metabolites as compared to normal EEG. Subsequent pathway enrichment analysis using MetaboAnalystR (all the results are shown in Table 1), revealed significant enrichment ($p < 0.01$) of several amino acid metabolic pathways (Fig. 18) in patients suffering from side effects. Whereas, only tyrosine metabolism was found to be significantly enriched ($p < 0.001$) in non-responders (Fig. 18). The association between downregulation of tyrosine metabolism and increased number of seizures (i.e. not responding adequately to medication) may be rationalized by the fact that neurotransmitter dopamine, which is known for its anti-epileptic action, is also downregulated, since it is synthesised from tyrosine and phenylalanine. Endogenous altered metabolites could be used to predict which patients are likely to respond to pharmacotherapy and to suffer from side effects.

Table 1 - part 1 - metabolites altered in side effect dataset.

| Compound | | Average | Identification | | |
|---|---|---|---|---|---|
| ID | Name | $log_2$ FC | m/z | m/z polarity | Adduct |
| C00213 | Sarcosine | 0.472 | 88.04032 | Neg | F |
| C00037 | Glycine | -0.061 | 74.02470 | Neg | F |
| C00033 | Acetate | -1.130 | 59.01378 | Neg | F |
| C03415 | N2-Succinyl-L-ornithine | 0.532 | 216.08653 | Pos | C |
| C00025 | L-Glutamic acid | 0.503 | 130.04994 131.03395 \| 148.06032 | Pos \| Pos \| Pos | E \| C \| A |
| C00026 | 2-Oxoglutarate | 0.431 ∦ | 145.01412 164.05530 | Neg \| Pos | F \| D |
| C00022 | Pyruvate | -0.067 | 8700873 \| .8.01201 | Neg \| Neg | F \| G |
| C00322 | 2-Oxoadipate | 0.189 ∦ | 143.03382 \| 159.02992 \| 161.04444 \| 178.07098 | Pos \| Neg \| Pos \| Pos | E \| F \| A \| D |
| C03793 √ | N6,N6,N6-Trimethyl-L-lysine | 0.722 | 171.14919 \| 172.13316 | Pos \| Pos | E \| C |
| C00232 | 4-Oxobutanoate | 0.328 ∦ | 101.02439 \| 102.02771 \| 103.03892 \| 120.06550 | Neg \| Neg \| Pos \| Pos | F \| G \| A \| D |
| C01879 √ | 5-Oxoproline | 0.363 ∦ | 112.03930 \| 113.02330 \| 128.03536 \| 130.04994 \| 147.07629 | Pos \| Pos \| Neg \| Pos \| Pos | E \| C \| F \| A \| D |
| C03239 √ | 6-Amino-2-oxohexanoate | 0.662 | 128.07063 \| 129.05463 \| 146.08106 \| 163.10771 | Pos \| Pos \| Pos \| Pos | E \| C \| A \| D |
| C00327 | Citrulline | 0.909 | 159.07646 | Pos | C |

(continued)

| Compound | | Average | Identification | | |
|---|---|---|---|---|---|
| ID | Name | $\log_2$ FC | m/z | m/z polarity | Adduct |
| C03912 | 1-Pyrroline-5-carboxylate | 0.708 | 112.04036 \| 114.05499 \| 131.08155 | Neg \| Pos \| Pos | F \| A \| D |
| C05545 √ | Protein N6,N6-dimethyl-L-lysine | 0.948 | 158.11758 | Pos | C |
| C05543 √ | 3-Dehydroxycarnitine | 0.674 | 128.10701 \| 129.09102 \| 146.11745 | Pos \| Pos \| Pos | E \| C \| A |
| C00077 √ | L-Ornithine | 0.912 | 116.07060 | Pos | C |
| CE4788 √ | acetamidopropanal | 0.915 | 116.07060 \| 117.07394 | Pos \| Pos | A \| B |
| C02630 √ | 2-Hydroxyglutarate | 0.203 ∦ | 131.03395 \| 147.02982 \| 166.07112 | Pos \| Neg \| Pos | E \| F \| D |
| C05572 | 4-Oxoglutaramate | 0.240 ∦ | 144.03016 \| 146.04467 | Neg \| Pos | F \| A |
| C00064 √ | L-Glutamine | 0.935 | 129.06587 \| 130.04994 \| 147.07629 | Pos \| Pos \| Pos | E \| C \| A |
| C03564 | 1-Pyrroline-2-carboxylate | 0.708 | 112.04036 \| 114.05499 \| 131.08155 | Neg \| Pos \| Pos | F \| A \| D |
| C00042 | Succinate | 0.147 ∦ | 101.02331 \| 117.01934 \| 118.02277 \| 136.06062 | Pos \| Neg \| Neg \| Pos | E \| F \| G \| D |
| C00047 √ | L-Lysine | 0.845 | 129.10223 \| 130.08632 | Pos \| Pos | E \| C |
| C00049 √ | L-Aspartic acid | 0.849 | 116.03422 \| 134.04489 | Pos \| Pos | E \| A |
| C00048 | Glyoxylate | 0.236 | 72.99307 \| 73.99641 | Neg \| Neg | F \| G |
| C02356 √ | (S)-2-Aminobutanoate | 0.955 | 104.07053 | Pos | A |
| C02946 √ | 4-Acetamidobutanoate | 0.662 | 128.07063 \| 129.05463 \| 146.08106 \| 163.10771 | Pos \| Pos \| Pos \| Pos | E \| C \| A \| D |
| C03711 | N-Methylphenylethanolamine | 0.572 | 135.08055 \| 152.10699 | Pos \| Pos | C \| A |
| C00547 √ | Arterenol | 0.526 ∦ | 152.07061 \| 153.05459 \| 170.08116 \| 187.10771 | Pos \| Pos \| Pos \| Pos | E \| C \| A \| D |
| C00763 √ | D-Proline | 0.915 | 116.07060 \| 117.07394 | Pos \| Pos | A \| B |
| C00956 | L-2-Aminoadipate | 0.562 | 144.06543 \| 145.04943 \| 162.07605 | Pos \| Pos \| Pos | E \| C \| A |
| C04092 √ | deltal-Piperideine-2-carboxylate | 0.579 | 110.06005 \| 111.04405 \| 126.05605 \| 128.07063 \| 145.09704 | Pos \| Pos \| Neg \| Pos \| Pos | E \| C \| F \| A \| D |
| C02238 √ | 5-Oxo-L-proline | 0.581 ∦ | 128.03536 \| 130.04994 \| 147.07629 | Neg \| Pos \| Pos | F \| A \| D |
| CE1936 | spermine dialdehyde | 1.321 | 186.14885 | Pos | C |
| CE1939 √ | spermidine monoaldehyde 1 | 0.953 | 130.12270 | Pos | C |
| C01029 √ | N8-Acetylspermidine | 0.528 | 171.14919 | Pos | C |
| C01035 | 4-Guanidinobutanoate | 0.984 | 129.06587 | Pos | C |
| C01250 √ | 2-Acetamido-5-oxopentanoate | 0.788 | 156.06544 \| 157.04959 \| 174.07612 \| 191.10258 | Pos \| Pos \| Pos \| Pos | E \| C \| A \| D |
| C05936 √ | N4-Acetylaminobutanal | 0.708 | 112.07568 \| 113.05969 \| 130.08632 | Pos \| Pos \| Pos | E \| C \| A |

(continued)

| Compound | | Average | Identification | | |
|---|---|---|---|---|---|
| ID | Name | $log_2$ FC | m/z | m/z polarity | Adduct |
| C00334 √ | GABA | 0.955 | 104.07053 | Pos | A |
| C00788 √ | L-Adrenaline | 0.663 ∦ | 166.08630 167.07027 \| 184.09687 \| 201.12342 | Pos \| Pos \| Pos \| Pos | E \| C \| A \| D |
| C00300 | Creatine | 0.438 | 115.05024 132.07680 | Pos \| Pos | C \| A |
| C00318 | L-Carnitine | 0.574 | 162.11244 163.11578 | Pos \| Pos | A \| B |
| C05947 √ | L-erythro-4-Hydroxyglutamate | 1.004 | 164.05530 | Pos | A |
| CE1940 √ | spermidine monoaldehyde 2 | 0.953 | 130.12270 | Pos | C |
| C00109 | 2-Oxobutanoate | 0.328 ∦ | 101.02439 \| 102.02771 \| 103.03892 \| 120.06550 | Neg \| Neg \| Pos \| Pos | F \| G \| A \| D |
| C01211 √ | Procollagen 5-hydroxy-L-lysine | 1.133 | 163.10771 | Pos | A |
| C00612 √ | N1-Acetylspermidine | 0.528 | 171.14919 | Pos | C |
| C00122 √ | Fumarate | 0.494 ∦ | 115.00370 \| 134.04489 | Neg \| Pos | F \| D |
| C01239 √ | N-Acetyl-beta-D-glucosaminylamine | 1.155 | 203.10264 \| 204.08659 | Pos \| Pos | E \| C |
| C01165 √ | L-Glutamate 5-semialdehyde | 0.291 ∦ | 114.05499 \| 115.03899 \| 132.06557 | Pos \| Pos \| Pos | E \| C \| A |
| C00450 √ | carboxylate delta1-Piperideine-6-L- | 0.579 | 110.06005 \| 111.04405 \| 126.05605 \| 128.07063 \| 145.09704 | Pos \| Pos \| Neg \| Pos \| Pos | E \| C \| F \| A \| D |
| C04281 √ | carboxylate L-1-Pyrroline-3-hydroxy-5- | 0.363 ∦ | 112.03930 \| 113.02330 \| 128.03536 \| 130.04994 \| 147.07629 | Pos \| Pos \| Neg \| Pos \| Pos | E \| C \| F \| A \| D |
| C04282 √ | carboxylate 1-Pyrroline-4-hydroxy-2- | 0.363 ∦ | 112.03930 \| 113.02330 \| 128.03536 \| 130.04994 \| 147.07629 | Pos \| Pos \| Neg \| Pos \| Pos | E \| C \| F \| A \| D |
| C00624 | N-Acetyl-L-glutamate | 0.716 | 172.06038 \| 190.07094 | Pos \| Pos | E \| A |
| C00152 √ | L-Asparagine | 0.803 | 115.05024 \| 116.03422 \| 133.06084 | Pos \| Pos \| Pos | E \| C \| A |
| C01157 √ | trans-4-Hydroxy-L-proline | 0.291 ∦ | 114.05499 \| 115.03899 \| 132.06557 | Pos \| Pos \| Pos | E \| C \| A |
| C02714 √ | N-Acetylputrescine | 0.704 | 114.09138 | Pos | C |
| C00148 √ | L-Proline | 0.915 | 116.07060 \| 117.07394 | Pos \| Pos | A \| B |
| C00437 √ | N-Acetylornithine | 1.348 | 157.09720 \| 158.08120 \| 175.10774 | Pos \| Pos \| Pos | E \| C \| A |
| C03440 | cis-4-Hydroxy-D-proline | 0.291 ∦ | 114.05499 \| 115.03899 \| 132.06557 | Pos \| Pos \| Pos | E \| C \| A |
| C05938 | semialdehyde L-4-Hydroxyglutamate | 0.503 | 130.04994 \| 131.03395 \| 148.06032 | Pos \| Pos \| Pos | E \| C \| A |
| C01602 √ | Ornithine | 0.912 | 116.07060 | Pos | C |
| C02735 | Phenylethanolamine | 0.754 | 138.09151 | Pos | A |
| C04076 √ | 2-Aminoadipate 6-semialdehyde | 0.662 | 128.07063 \| 129.05463 \| 146.08106 \| 163.10771 | Pos \| Pos \| Pos \| Pos | E \| C \| A \| D |

(continued)

| Compound | | Average | Identification | | |
|---|---|---|---|---|---|
| ID | Name | $log_2$ FC | m/z | m/z polarity | Adduct |
| C00989 √ | 4-Hydroxybutanoate | 1.113 | 103.04007 \| 104.04342 \| 122.08114 | Neg \| Neg \| Pos | F \| G \| D |
| C00408 √ | L-Pipecolate | 0.708 | 112.07568 \| 113.05969 \| 130.08632 | Pos \| Pos \| Pos | E \| C \| A |
| C00402 √ | D-Aspartate | 0.849 | 116.03422 \| 134.04489 | Pos \| Pos | E \| A |

Table 1 continued - part 2 - metabolites altered in drug response dataset.

| Compound | | Average | | Identification | |
|---|---|---|---|---|---|
| ID | Name | $log_2$ FC | m/z | m/z polarity | Adduct |
| C05589 | L-Normetanephrine | -0.654 | 166.08630 \| 167.07027 \| 184.09687 \| 201.12342 | Pos \| Pos \| Pos \| Pos | E \| C \| A \| D |
| C05588 √ | L-Metanephrine | -0.936 | 180.10186 \| 181.08587 \| 198.11255 | Pos \| Pos \| Pos | E \| C \| A |
| C05581 √ | 3-Methoxy-4-hydroxyphenylacetaldehyde | -0.818 | 149.05967 \| 167.07027 \| 184.09687 | Pos \| Pos \| Pos | E \| A \| D |
| C05583 | 3-Methoxy-4-hydroxyphenylglycolaldehyde | -0.668 | 165.05467 \| 183.06522 \| 200.09175 | Pos \| Pos \| Pos | E \| A \| D |
| C05582 | Homovanillate | -0.668 | 165.05467 \| 183.06522 \| 200.09175 | Pos \| Pos \| Pos | E \| A \| D |
| C05584 | Vanillylmandelicacid | -0.190 | 216.08653 | Pos | D |
| C05587 √ | 3-Methoxytyramine | -0.743 | 150.09136 \| 151.07538 \| 168.10193 | Pos \| Pos \| Pos | E \| C \| A |
| C07086 | Phenylacetate | -0.543 | 135.04534 137.05993 154.08622 | Neg \| Pos \| Pos | F \| A \| D |
| C04043 | Protocatechuatealdehy de | -0.558 | 135.04417 151.04003 153.05459 \| 170.08116 | Pos \| Neg \| Pos \| Pos | E \| F \| A \| D |
| C00025 | L-Glutamic acid | -0.277 ∦ | 130.04994 \| 131.03395 148.06032 | Pos \| Pos \| Pos | E \| C \| A |
| C00026 | 2-Oxoglutarate | -0.449 | 145.01412 \| 164.05530 | Neg \| Pos | F \| D |
| C00022 | Pyruvate | 0.215 | 87.00873 \| .8.01201 | Neg \|Neg | F \| G |
| CE4888 | dopaminochrome | -0.469 | 148.04036 \| 150.05498 | Neg Pos | F \| A |
| C02442√ | N-Methyltyramine | -0.470 | 135.08055 \| 152.10699 | Pos \| Pos | C \| A |
| CE4890 √ | N-methylsalsolinol | -0.849 | 177.09099 \| 194.11763 | Pos \| Pos | C \| A |

(continued)

| Compound | | Average | | Identification | |
|---|---|---|---|---|---|
| ID | Name | $log_2$ FC | m/z | m/z polarity | Adduct |
| C00079 | L-Phenylalanine | -0.831 | 149.05967 \| 166.08630 | Pos \| Pos | C \| A |
| C00072 | Ascorbate | -0.549 | 194.06603 | Pos | D |
| C05578 | DHI | -0.469 | 148.04036 \| 150.05498 | Neg \| Pos | F \| A |
| C05579 √ | Indole-5,6-quinone | -0.350 | 146.02472 \| 165.06591 | Neg \| Pos | F \| D |
| C05576 | 3,4-Dihydroxyphenylethyleneglycol | -0.416 ⫳ | 153.05459 \| 169.05062 \| 171.06516 \| 188.09174 | Pos \| Neg \| Pos \| Pos | E \| F \| A \| D |
| C05577 | 3,4-Dihydroxymandelaldehyde | -0.587 | 151.03900 \| 167.03523 \| 169.04953 \| 186.07614 | Pos \| Neg \| Pos \| Pos | E \| F \| A \| D |
| C00064 | L-Glutamine | 0.025 ⫳ | 129.06587 \| 130.04994 \| 147.07629 | Pos \| Pos \| Pos | E \| C \| A |
| CE5538 | noradrenochrome | -0.404 | 166.04990 | Pos | A |
| C03758 √ | Dopamine | -0.615 | 136.07587 \| 137.05993 \| 154.08622 \| 171.11280 | Pos \| Pos \| Pos \| Pos | E \| C \| A \| D |
| C05332 √ | Phenethylamine | -0.904 | 105.06988 \| 122.09639 | Pos \| Pos | C \| A |
| C06199 | Hordenine | -0.567 | 149.09605 \| 166.12265 | Pos \| Pos | C \| A |
| C00041 | L-Alanine | -0.068 | 88.04032 | Neg | F |
| C00547 | Arterenol | -0.556 | 152.07061 \| 153.05459 \| 170.08116 \| 187.10771 | Pos \| Pos \| Pos \| Pos | E \| C \| A \| D |
| C00544 | Homogentisate | -0.587 | 151.03900 \| 167.03523 \| 169.04953 \| 186.07614 | Pos \| Neg \| Pos \| Pos | E \| F \| A \| D |
| CE5542 | noradrenochrome o-semiquinone | -1.222 | 167.05769 | Pos | A |
| C00082 | L-Tyrosine | -0.709 | 164.07056 \| 165.05467 \| 182.08125 | Pos \| Pos \| Pos | E \| C \| A |
| C09642 √ | Salsolinol | -1.283 | 163.07531 \| 180.10186 \| 181.10524 | Pos \| Pos \| Pos | C \| A \| B |
| C02218 | 2-Aminoacrylate | 0.301 | 86.02478 | Neg | F |
| C09640 √ | (-)-Salsoline | -0.849 | 177.09099 \| 194.11763 | Pos \| Pos | C \| A |
| C00788 | L-Adrenaline | -0.654 | 166.08630 \| 167.07027 \| 184.09687 \| 201.12342 | Pos \| Pos \| Pos \| Pos | E \| C \| A \| D |
| C00601 | Phenylacetaldehyde | -0.438 | 138.09151 | Pos | D |

(continued)

| Compound | | Average | | Identification | |
|---|---|---|---|---|---|
| ID | Name | $log_2$ FC | m/z | m/z polarity | Adduct |
| C07453 √ | Epinine | -0.743 | 150.09136 \| 151.07538 \| 168.10193 | Pos \| Pos \| Pos | E \| C \| A |
| C00483 | Tyramine | -0.438 | 138.09151 | Pos | A |
| C00122 | Fumarate | -0.488 ⫳ | 115.00370 134.04489 | Neg \| Pos | F \| D |
| C02505 √ | 2-Phenylacetamide | -0.747 | 118.06507 136.07587 \| 137.07930 | Pos \| Pos \| Pos | E \| A \| B |
| C02763 | enol-Phenylpyruvate | -0.725 | 147.04394 165.05467 \| 182.08125 | Pos \| Pos \| Pos | E \| A \| D |
| CE2176 | 3-O-methyldopa | -0.761 | 195.06529 | Pos | C |
| CE2172 √ | 6,7-dihydroxy-1,2,3,4-THIQ | -0.831 | 149.05967 \| 166.08630 | Pos \| Pos | C \| A |
| CE2173 | N-methyl-4,6,7-trihydroxy-1,2,3,4-THIQ | -1.121 | 179.07023 \| 196.09692 | Pos \| Pos | C \| A |
| C00642 | 4-Hydroxypheirylacetate | -0.558 | 135.04417 \| 151.04003 \| 153.05459 \| 170.08116 | Pos \| Neg \| Pos \| Pos | E \| F \| A \| D |
| CE5629 | 1,2-dehydrosalsolinol | -0.748 | 161.05971 | Pos | C |
| CE2174 | 4,6,7-trihydroxy-1,2,3,4-THIQ | -0.709 | 164.07056 \| 165.05467 182.08125 | Pos \| Pos \| Pos | E C A |
| C00164 | Acetoacetate | -0.162 ⫳ | 101.02439 \| 10202771 103.03892 \| 120.06550 | Neg \| Neg \| Pos \| Pos | F G A \| D |
| C00166 | Phenylpyruvate | -0.725 | 147.04394 165.05467 182.08125 | Pos \| Pos \| Pos | E A D |
| C05852 | 2-Hydroxypheirylacetate | -0.558 | 135.04417 151.04003 153.05459 \| 170.08116 | Pos Neg Pos \| Pos | E F A \| D |
| C01161 | Homoprotocatechuate | -0.587 | 151.03900 167.03523 169.04953 \| 186.07614 | Pos Neg Pos \| Pos | E F A \| D |
| C03765 | 4-Hydroxypheirylacetaldehyde | -0.543 | 135.04534 137.05993 154.08622 | Neg \| Pos \| Pos | F A D |
| C05594 | 3-Methoxy-4-hydroxyphenylethyleneglycol | -0.534 | 167.07027 \| 183.06627 \| 185.08097 \| 202.10738 | Pos \| Neg \| Pos \| Pos | E \| F \| A \| D |

## Claims

**1.** A method for therapeutic drug monitoring, the method comprising:

(a) providing a sample of breath exhaled by the subject to a mass spectrometer;
(b) collecting the mass spectra of substances present in the exhaled breath in positive and/or negative mode;
(c) analysing the mass spectra using a previously trained mathematical model; and
(d) determining the risk estimate of side effects of a drug and/or probability estimate of drug response in the subject based on the analysis of the mass spectra of the exhaled breath;

wherein the previously trained mathematical model relies, as predictors, on the signal intensity or area under the curve of selected m/z peaks in the mass spectra that are due to metabolites whose concentration is modulated by administration of the drug.

**2.** The method for therapeutic drug monitoring according to claim 1, wherein the contribution of different metabolic routes in metabolizing the therapeutic drug is calculated based on the ratios of values of the predictors.

**3.** The method of claim 1 or 2, wherein the therapeutic drug is a substance used as an anti-epileptic medication.

**4.** The method of claim 3, wherein the antiepileptic medication is valproate.

**5.** The method of claim 1 or 2, wherein the therapeutic drug is a substance used as an anti-cancer medication.

**6.** The method of claim 5, wherein the anti-cancer medication is methotrexate.

**7.** The method of any one of claims 1 to 6, wherein the previously trained mathematical model is Gaussian process regression.

**8.** An apparatus for use in the method of any one of the preceding claims, the apparatus comprising

(a) a disposable mouthpiece for collecting a sample of breath exhaled by the subject;
(b) a connector for delivering the collected sample to the ionisation device;
(c) a mass spectrometer comprising an electrospray ionisation module and a detection module;
(d) a computer interface for analysis and determination of the risk estimate of side effects of a drug and/or probability estimate of drug response in the subject based on the analysis of the mass spectra of the exhaled breath;

wherein the apparatus is configured for carrying out a previously trained mathematical model used in the determination of the risk estimate of side effects of a drug and/or probability estimate of drug response in the subject based on the analysis of the mass spectra of the exhaled breath, wherein the mathematical model relies, as predictors, on the signal intensity or area under the curve of selected m/z peaks in the mass spectra that are due to metabolites whose concentration is modulated by administration of the drug, and wherein the mass spectrometer is calibrated for analyzing breath samples.

**9.** The apparatus of claim 8, wherein the connector is configured for optimal delivery of compounds that give raise to m/z in the range of 50-1000.

**10.** The apparatus of claim 8 or 9, wherein the apparatus is configured to perform the analysis in real time.

**11.** The apparatus of any one of claims 8 to 10, wherein the computer interface is configured to perform a quality control of the sample analysis based on the variability between repeated measurements and measurements with standardized gas composition, preferably containing compounds that give raise to m/z in the range of 100-200.

**Patentansprüche**

**1.** Verfahren zur Überwachung therapeutischer Arzneimittel (Therapeutisches Drug Monitoring), wobei das Verfahren umfasst:

(a) Bereitstellen einer Probe des vom Individuum ausgeatmeten Atems für ein Massenspektrometer;
(b) Erfassen der Massenspektren der in dem ausgeatmeten Atem vorhandenen Substanzen im positiven und/oder negativen Modus;

(c) Analysieren der Massenspektren unter Verwendung eines zuvor trainierten mathematischen Modells; und
(d) Bestimmen der Risikoabschätzung der Nebenwirkungen eines Arzneimittels und/oder der Wahrscheinlichkeitsschätzung des Ansprechens auf das Arzneimittel bei dem Individuum auf der Grundlage der Analyse der Massenspektren des ausgeatmeten Atems;

wobei sich das zuvor trainierte mathematische Modell als Prädiktoren auf die Signalintensität oder die Fläche unter der Kurve ausgewählter m/z-Peaks in den Massenspektren stützt, die auf Metaboliten zurückzuführen sind, deren Konzentration durch die Verabreichung des Arzneimittels moduliert wird.

**2.** Verfahren zur Überwachung therapeutischer Arzneimittel (Therapeutisches Drug Monitoring) nach Anspruch 1, wobei der Beitrag verschiedener Stoffwechselwege zur Metabolisierung des therapeutischen Arzneimittels auf der Grundlage der Verhältnisse der Werte der Prädiktoren berechnet wird.

**3.** Verfahren nach Anspruch 1 oder 2, wobei das therapeutische Arzneimittel eine Substanz ist, die als Antiepileptikum verwendet wird.

**4.** Verfahren nach Anspruch 3, wobei das Antiepileptikum Valproat ist.

**5.** Verfahren nach Anspruch 1 oder 2, wobei das therapeutische Arzneimittel eine Substanz ist, die als Antikrebsmittel verwendet wird.

**6.** Verfahren nach Anspruch 5, wobei das Antikrebsmittel Methotrexat ist.

**7.** Verfahren nach einem der Ansprüche 1 bis 6, wobei das zuvor trainierte mathematische Modell eine Gauß-Prozess-Regression ist.

**8.** Vorrichtung zur Verwendung bei dem Verfahren nach einem der vorangehenden Ansprüche, wobei die Vorrichtung umfasst

(a) ein Einweg-Mundstück zum Gewinnen einer Probe des vom Individuum ausgeatmeten Atems;
(b) einen Anschluss zur Abgabe der entnommenen Probe an die Ionisationsvorrichtung;
(c) ein Massenspektrometer, das ein Elektrospray-Ionisationsmodul und ein Detektionsmodul umfasst;
(d) eine Computerschnittstelle zur Analyse und Bestimmung der Risikoabschätzung von Nebenwirkungen eines Arzneimittels und/oder der Wahrscheinlichkeitsschätzung des Ansprechens auf das Arzneimittel bei dem Individuum auf der Grundlage der Analyse der Massenspektren des ausgeatmeten Atems;

wobei die Vorrichtung konfiguriert ist, ein zuvor trainiertes mathematisches Modell auszuführen, das zur Bestimmung der Risikoabschätzung von Nebenwirkungen eines Arzneimittels und/oder der Wahrscheinlichkeitsschätzung des Ansprechens auf das Arzneimittel bei dem Individuum auf der Grundlage der Analyse der Massenspektren des ausgeatmeten Atems verwendet wird, wobei sich das mathematische Modell als Prädiktoren auf die Signalintensität oder die Fläche unter der Kurve ausgewählter m/z-Peaks in den Massenspektren stützt, die auf Metaboliten zurückzuführen sind, deren Konzentration durch die Verabreichung des Arzneimittels moduliert wird, und wobei das Massenspektrometer für die Analyse von Atemproben kalibriert ist.

**9.** Vorrichtung nach Anspruch 8, wobei der Anschluss für die optimale Abgabe von Verbindungen konfiguriert ist, die m/z-Werte im Bereich von 50 bis 1000 ergeben.

**10.** Vorrichtung nach Anspruch 8 oder 9, wobei die Vorrichtung konfiguriert ist, die Analyse in Echtzeit durchzuführen.

**11.** Vorrichtung nach einem der Ansprüche 8 bis 10, wobei die Computerschnittstelle konfiguriert ist, eine Qualitätskontrolle der Probenanalyse auf der Grundlage der Variabilität zwischen wiederholten Messungen und Messungen mit standardisierter Gaszusammensetzung durchzuführen, bevorzugt enthaltend Verbindungen, die m/z-Werte im Bereich von 100 bis 200 ergeben.

## Revendications

**1.** Procédé de suivi thérapeutique pharmacologique, le procédé comprenant :

(a) fournir un échantillon d'haleine expirée par le sujet à un spectromètre de masse ;
(b) collecter les spectres de masse de substances présentes dans l'haleine expirée en mode positif et/ou négatif ;
(c) analyser les spectres de masse à l'aide d'un modèle mathématique préalablement entraîné ; et
(d) déterminer l'estimation du risque d'effets secondaires d'un médicament et/ou l'estimation de la probabilité de réponse à un médicament chez le sujet sur la base de l'analyse du spectre de masse de l'haleine expirée ;

dans lequel le modèle mathématique préalablement entraîné repose, comme prédicteurs, sur l'intensité du signal ou l'aire sous la courbe de pics m/z sélectionnés dans les spectres de masse qui sont dus à des métabolites dont la concentration est modulée par l'administration du médicament.

**2.** Procédé de suivi thérapeutique pharmacologique selon la revendication 1, dans lequel la contribution de différentes voies métaboliques dans la métabolisation du médicament thérapeutique est calculée sur la base des rapports de valeurs des prédicteurs.

**3.** Procédé selon la revendication 1 ou 2, dans lequel le médicament thérapeutique est une substance utilisée comme médicament anti-épileptique.

**4.** Procédé selon la revendication 3, dans lequel le médicament antiépileptique est le valproate.

**5.** Procédé selon la revendication 1 ou 2, dans lequel le médicament thérapeutique est une substance utilisée comme médicament anticancéreux.

**6.** Procédé selon la revendication 5, dans lequel le médicament anticancéreux est le méthotrexate.

**7.** Procédé selon l'une quelconque des revendications 1 à 6, dans lequel le modèle mathématique préalablement entraîné est une régression de processus gaussien.

**8.** Appareil destiné à être utilisé dans le procédé selon l'une quelconque des revendications précédentes, l'appareil comprenant

(a) un embout buccal jetable pour collecter un échantillon d'haleine expirée par le sujet ;
(b) un raccord pour délivrer l'échantillon prélevé au dispositif d'ionisation ;
(c) un spectromètre de masse comprenant un module d'ionisation electrospray et un module de détection ;
(d) une interface informatique pour l'analyse et la détermination de l'estimation du risque d'effets secondaires d'un médicament et/ou de l'estimation de la probabilité de réponse à un médicament chez le sujet, sur la base de l'analyse du spectre de masse de l'haleine expirée ;

dans lequel l'appareil est configuré pour exécuter un modèle mathématique préalablement entraîné utilisé dans la détermination de l'estimation du risque d'effets secondaires d'un médicament et/ou de l'estimation de la probabilité de réponse à un médicament chez le sujet sur la base de l'analyse des spectres de masse de l'haleine expirée, le modèle mathématique reposant, comme prédicteurs, sur l'intensité du signal ou l'aire sous la courbe des pics m/z sélectionnés dans les spectres de masse qui sont dus à des métabolites dont la concentration est modulée par l'administration du médicament, et dans lequel le spectromètre de masse est étalonné pour analyser des échantillons d'haleine.

**9.** Appareil selon la revendication 8, dans lequel le connecteur est configuré pour une délivrance optimale de composés qui donnent une élévation de m/z dans la plage de 50 à 1 000.

**10.** Appareil selon la revendication 8 ou 9, dans lequel l'appareil est configuré pour effectuer l'analyse en temps réel.

**11.** Appareil selon l'une quelconque des revendications 8 à 10, dans lequel l'interface informatique est configurée pour effectuer un contrôle qualité de l'analyse d'échantillon basé sur la variabilité entre des mesures répétées et des mesures avec une composition de gaz normalisée, contenant de préférence des composés qui donnent une élévation de m/z dans la plage de 100 à 200.

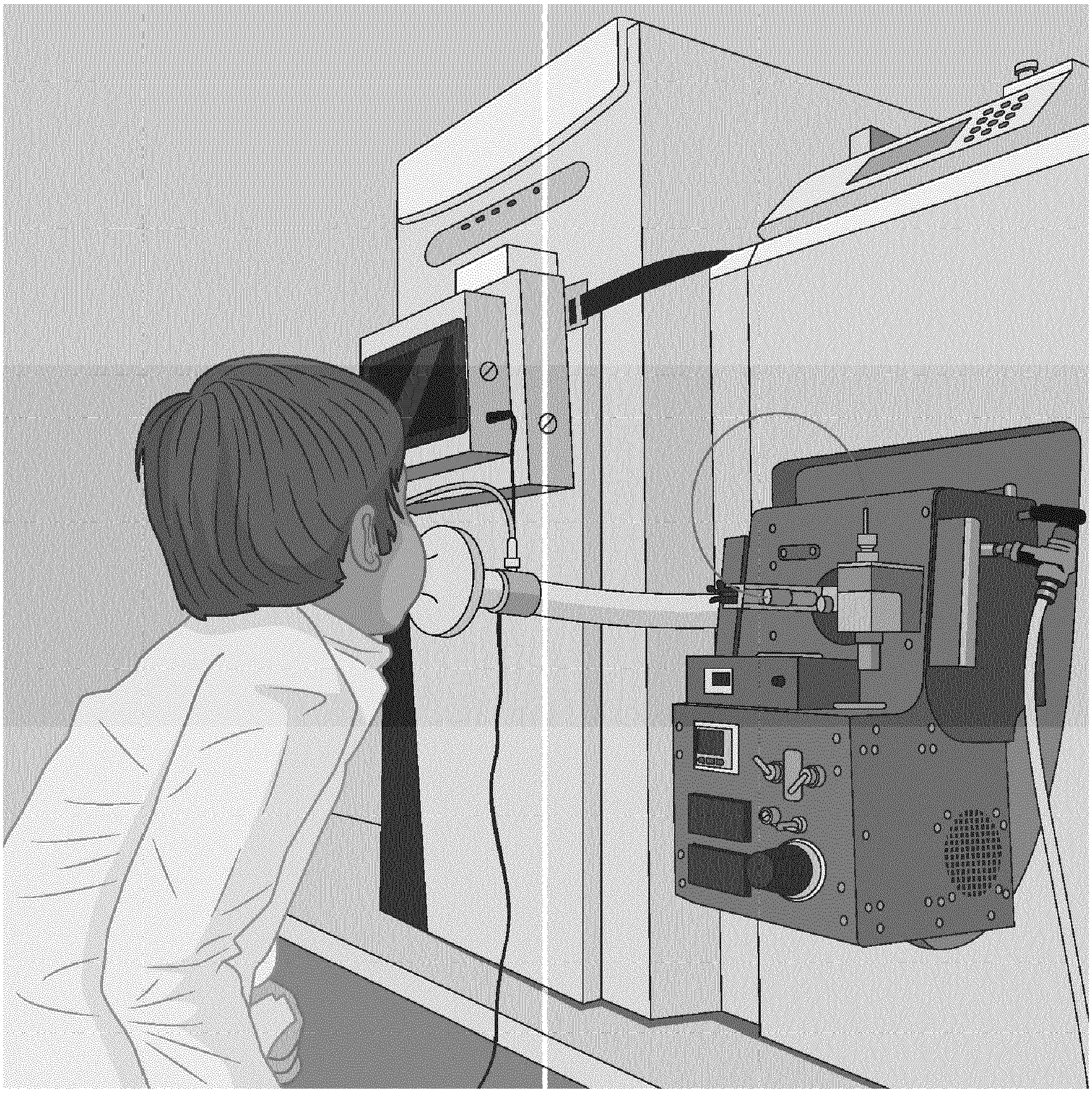

Figure 1.

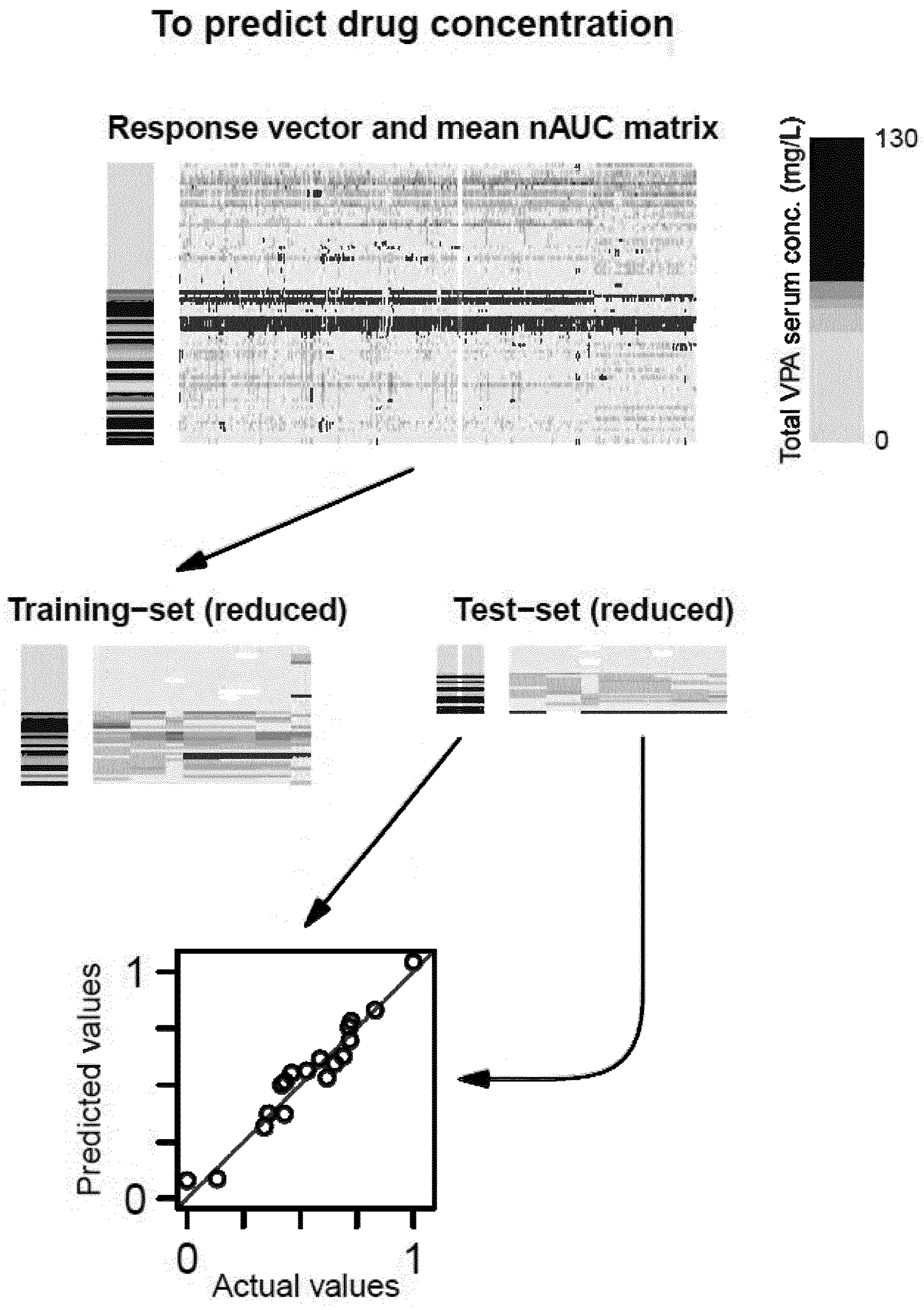
To predict drug concentration
Response vector and mean nAUC matrix
Total VPA serum conc. (mg/L)
130
0
Training−set (reduced)
Test−set (reduced)
Predicted values
1
0
0
1
Actual values

Figure 2.

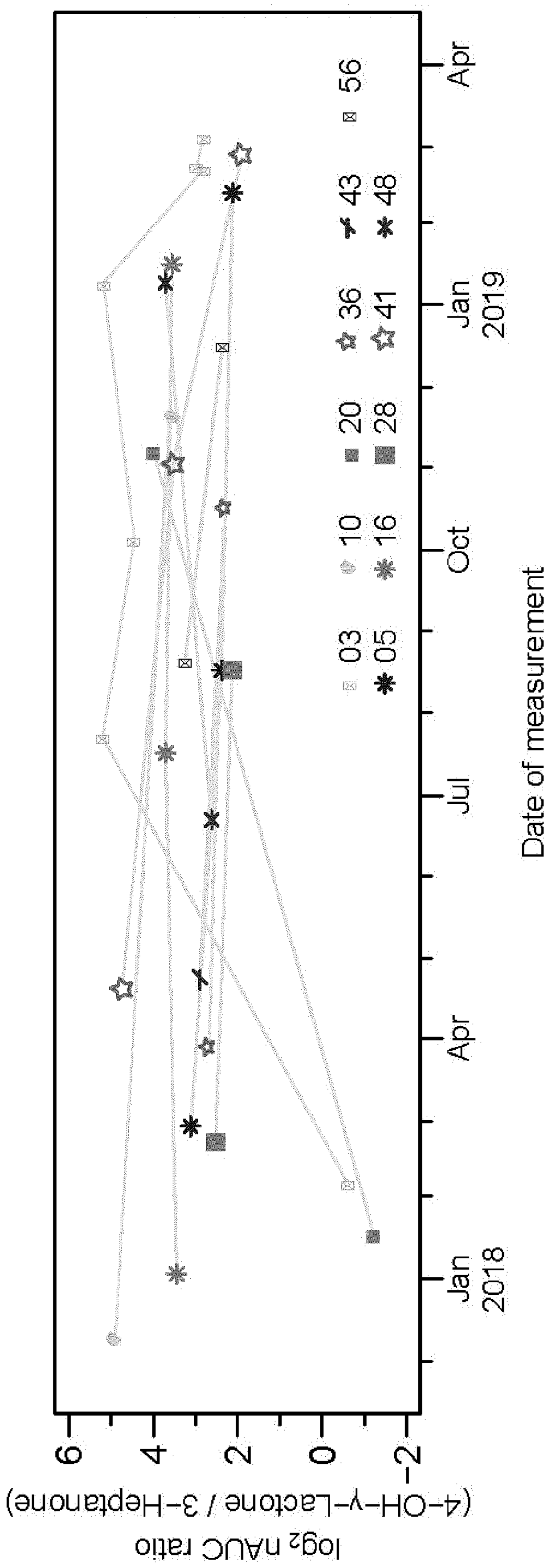
log2 nAUC ratio
(4−OH−γ−Lactone / 3−Heptanone)
6
4
2
0
−2
Jan
2018
Apr
Jul
Oct
Jan
2019
Apr
Date of measurement
03
05
10
16
20
28
36
41
43
48
56

Figure 3.

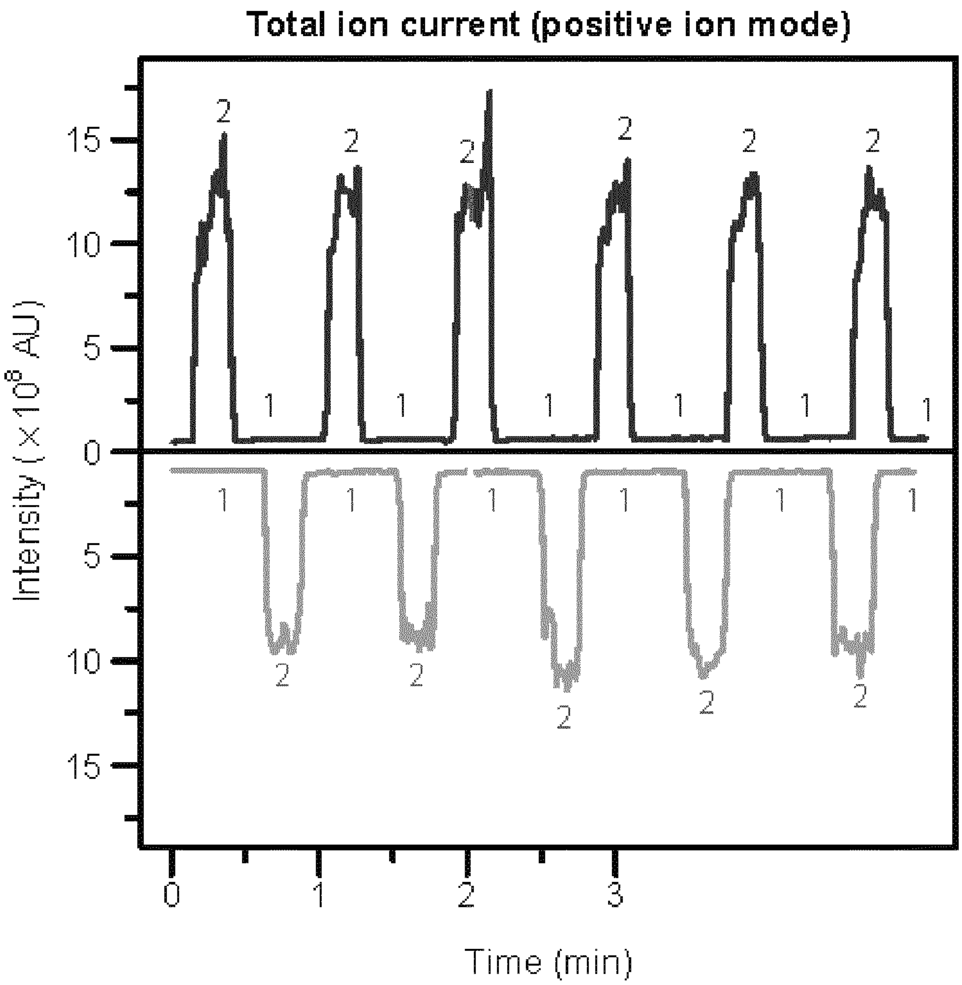
Total ion current (positive ion mode)
Intensity ( ×10$^8$ AU)
Time (min)
0
1
2
3
0
5
10
15
2
1

Figure 4.

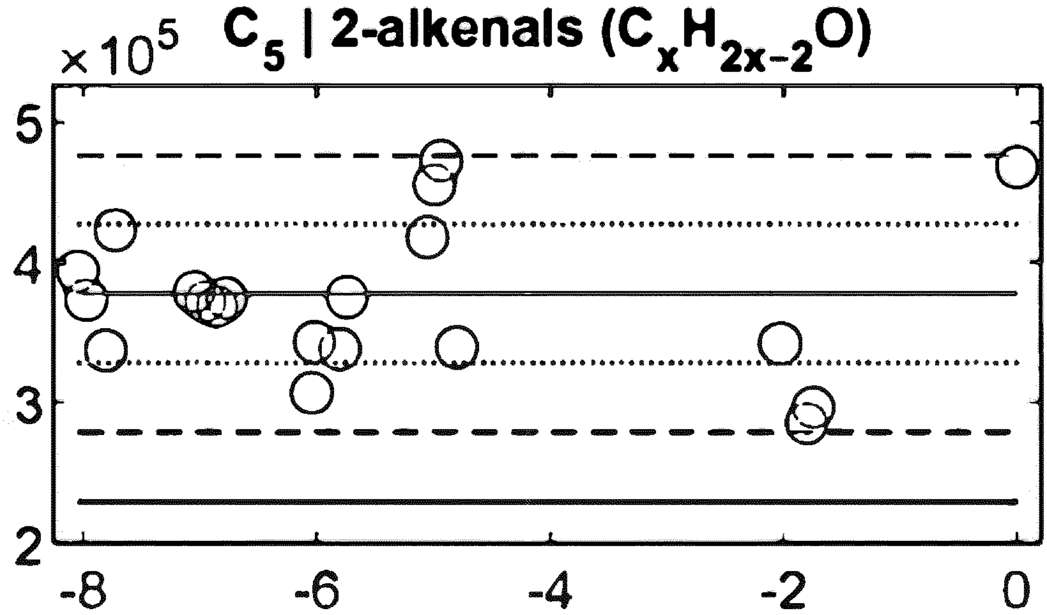


Figure 5.

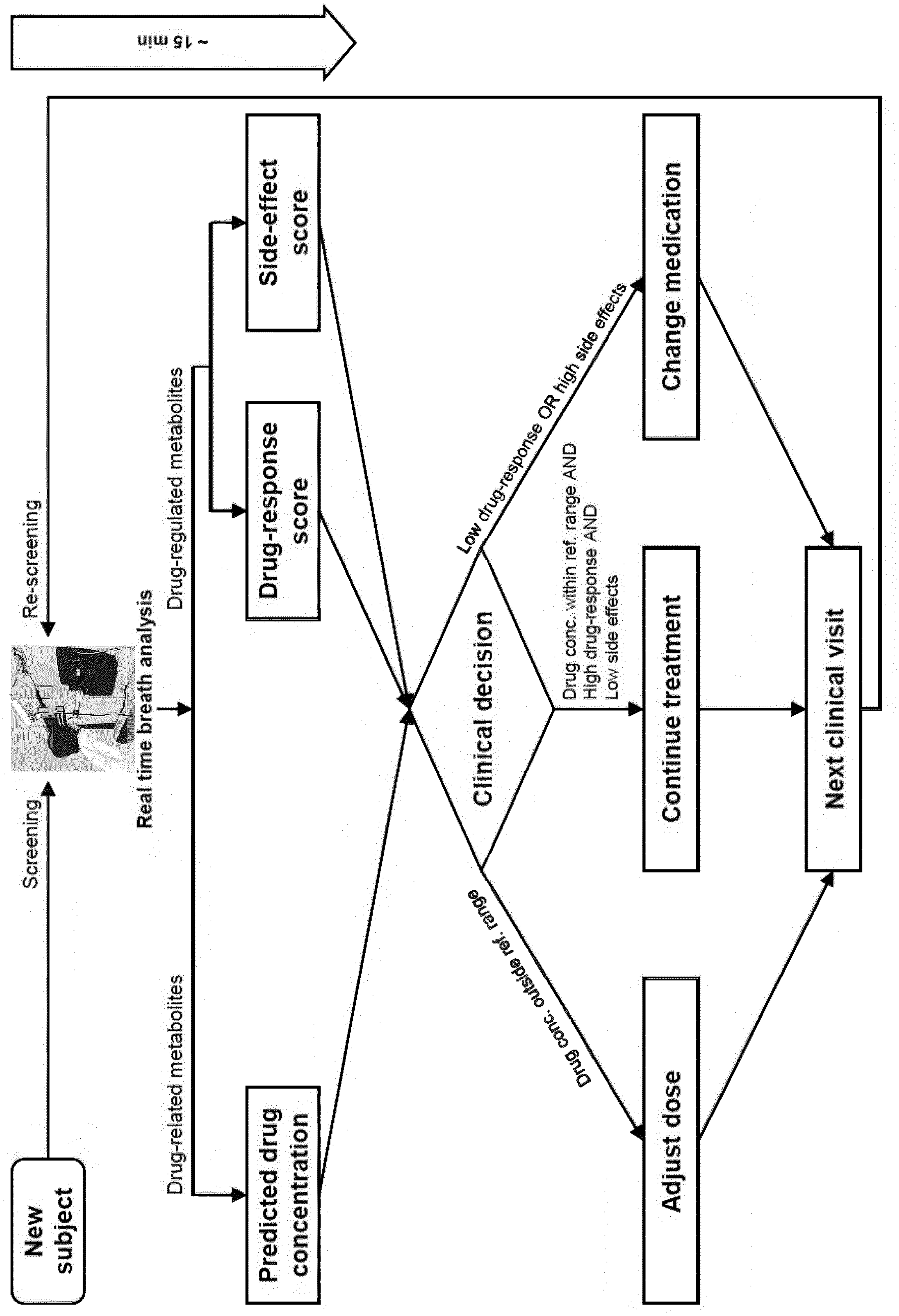
~ 15 min
New subject
Screening
Re-screening
Real time breath analysis
Drug-related metabolites
Drug-regulated metabolites
Predicted drug concentration
Drug-response score
Side-effect score
Clinical decision
Low drug-response OR high side effects
Drug conc. within ref. range AND
High drug-response AND
Low side effects
Drug conc. outside ref. range
Change medication
Continue treatment
Adjust dose
Next clinical visit

Figure 6.

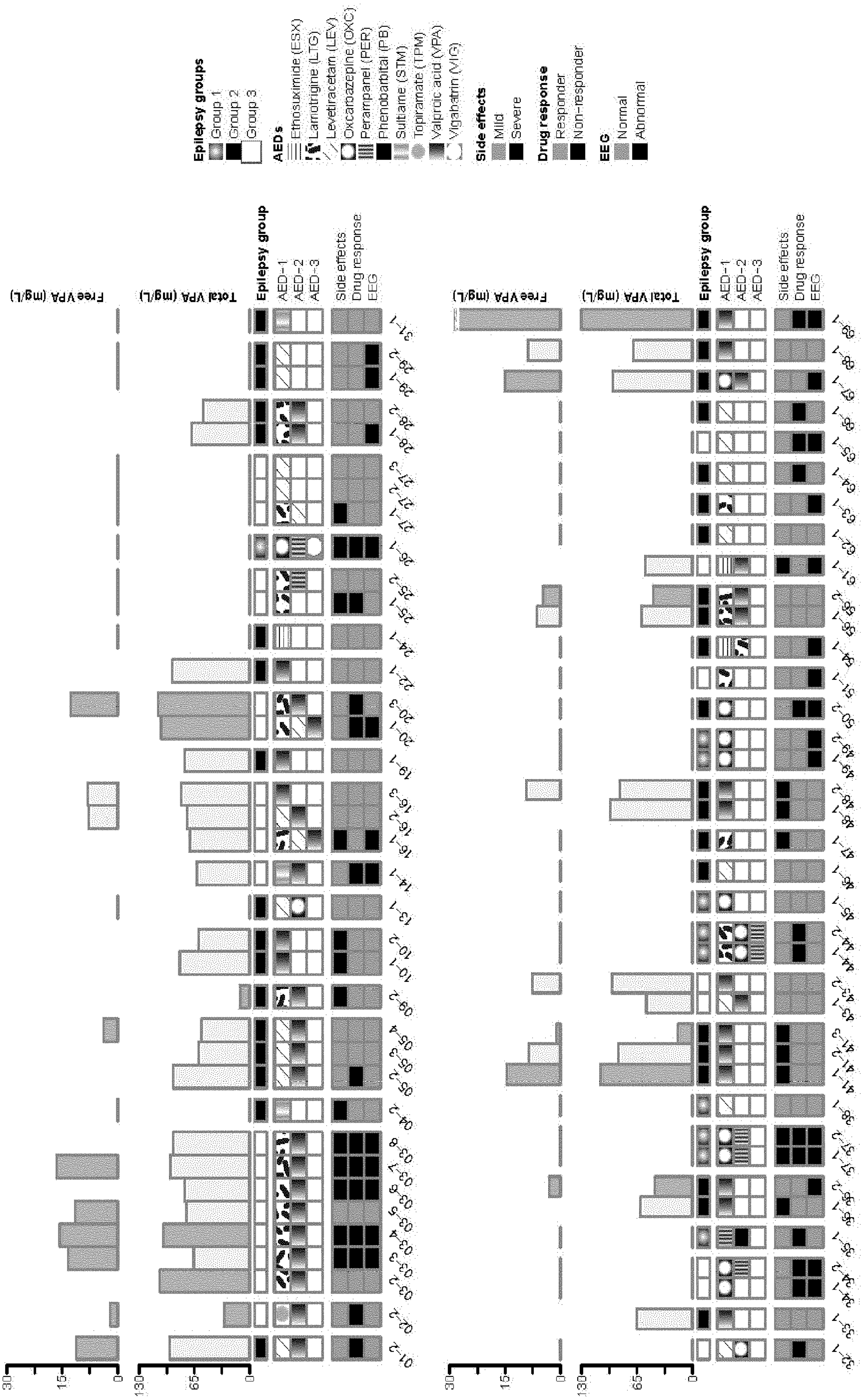

Epilepsy groups
Group 1
Group 2
Group 3
AEDs
Ethosuximide (ESX)
Lamotrigine (LTG)
Levetiracetam (LEV)
Oxcarbazepine (OXC)
Perampanel (PER)
Phenobarbital (PB)
Sultiame (STM)
Topiramate (TPM)
Valproic acid (VPA)
Vigabatrin (VIG)
Side effects
Mild
Severe
Drug response
Responder
Non-responder
EEG
Normal
Abnormal
Free VPA (mg/L)
Total VPA (mg/L)
Epilepsy group
AED-1
AED-2
AED-3
Side effects
Drug response
EEG


Figure 7.

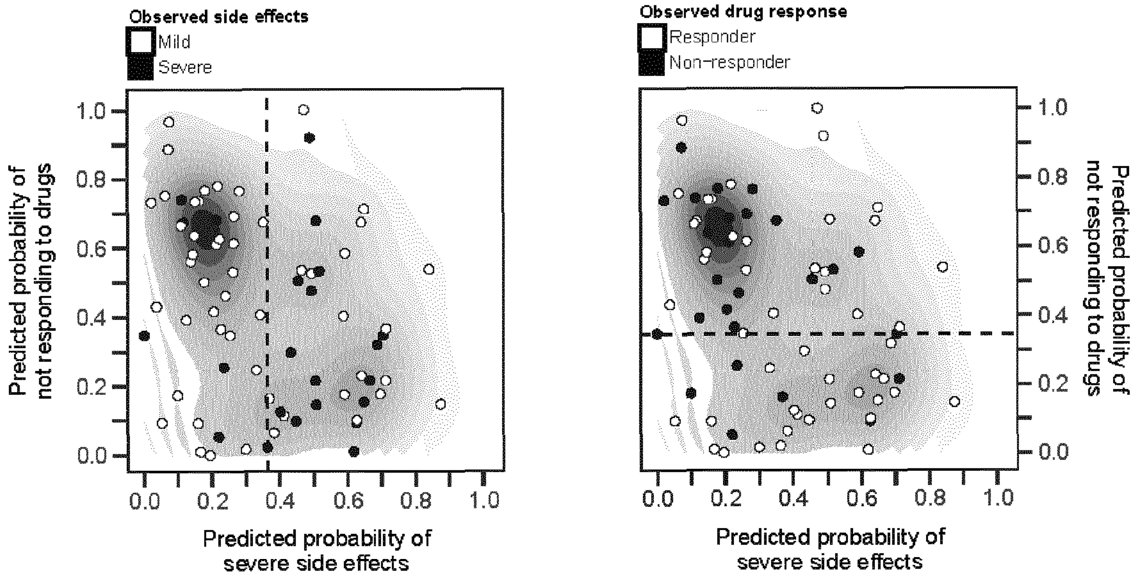
Observed side effects
Mild
Severe
Predicted probability of not responding to drugs
Predicted probability of severe side effects
1.0
0.8
0.6
0.4
0.2
0.0
Observed drug response
Responder
Non-responder

Figure 8.

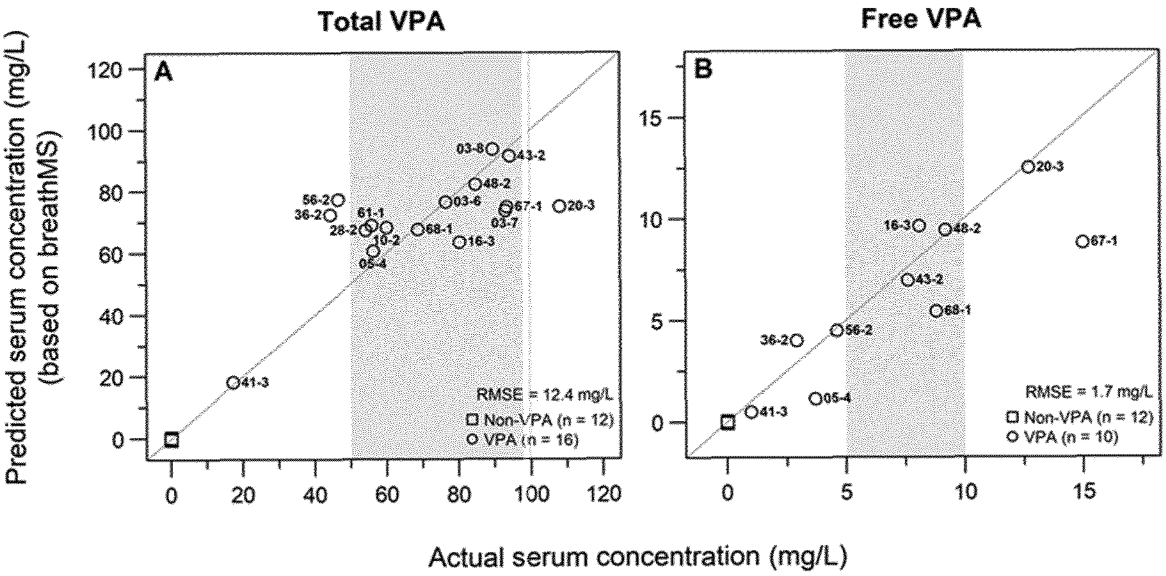
Total VPA
Free VPA
A
B
Predicted serum concentration (mg/L)
(based on breathMS)
Actual serum concentration (mg/L)
03-8
43-2
48-2
56-2
36-2
61-1
03-6
67-1
20-3
03-7
28-2
10-2
68-1
16-3
05-4
41-3
RMSE = 12.4 mg/L
Non-VPA (n = 12)
VPA (n = 16)
16-3
48-2
67-1
43-2
68-1
36-2
56-2
05-4
41-3
RMSE = 1.7 mg/L
Non-VPA (n = 12)
VPA (n = 10)

Figure 9.

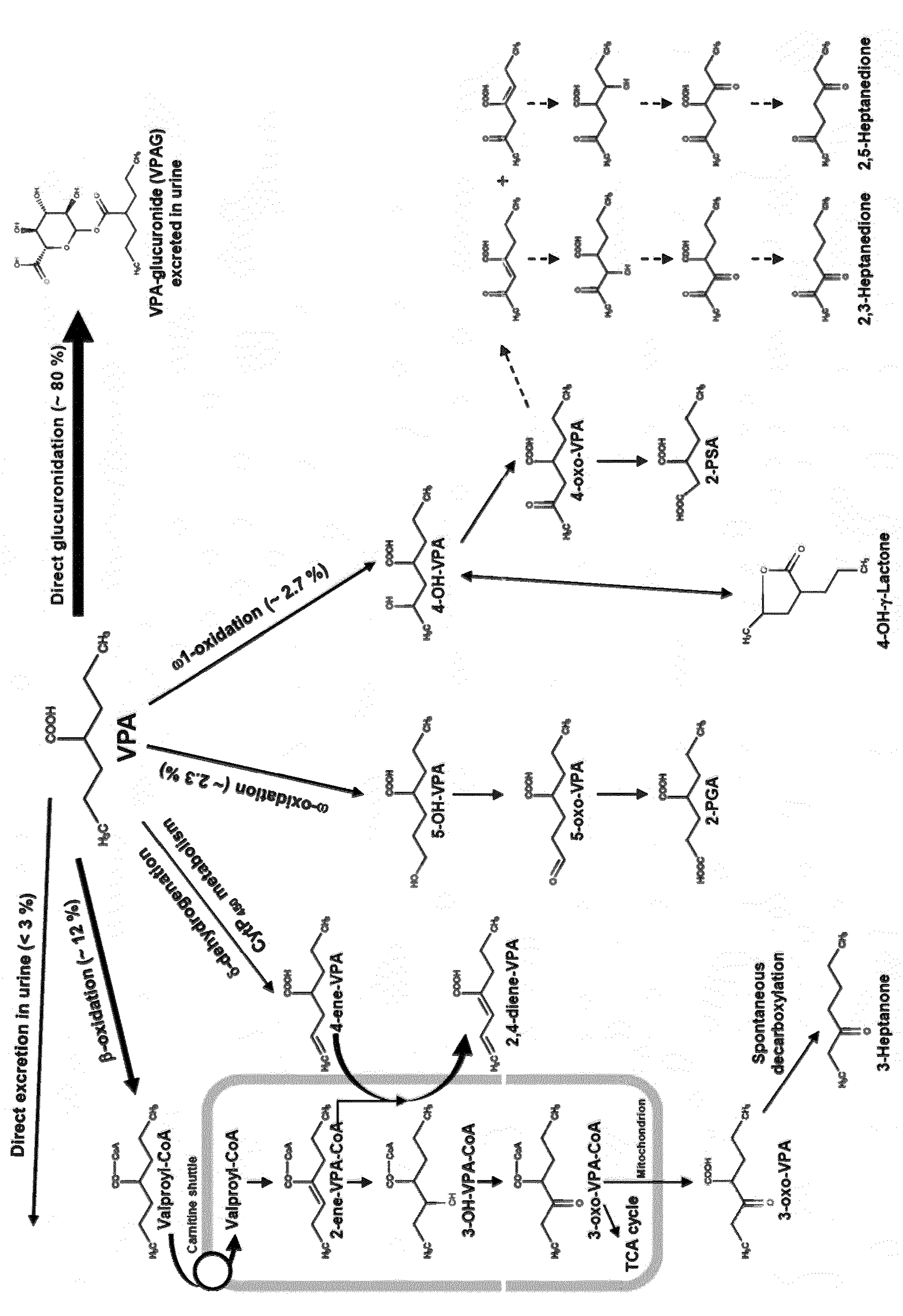
Direct glucuronidation (~ 80 %)
VPA-glucuronide (VPAG) excreted in urine
VPA
ω1-oxidation (~ 2.7 %)
4-OH-VPA
4-oxo-VPA
2-PSA
4-OH-γ-Lactone
2,3-Heptanedione
2,5-Heptanedione
ω-oxidation (~ 2.3 %)
5-OH-VPA
5-oxo-VPA
2-PGA
Cyt P450 metabolism
δ-dehydrogenation
4-ene-VPA
2,4-diene-VPA
Direct excretion in urine (< 3 %)
β-oxidation (~ 12 %)
Valproyl-CoA
Carnitine shuttle
Valproyl-CoA
2-ene-VPA-CoA
3-OH-VPA-CoA
3-oxo-VPA-CoA
Mitochondrion
TCA cycle
3-oxo-VPA
Spontaneous decarboxylation
3-Heptanone

Figure 10.

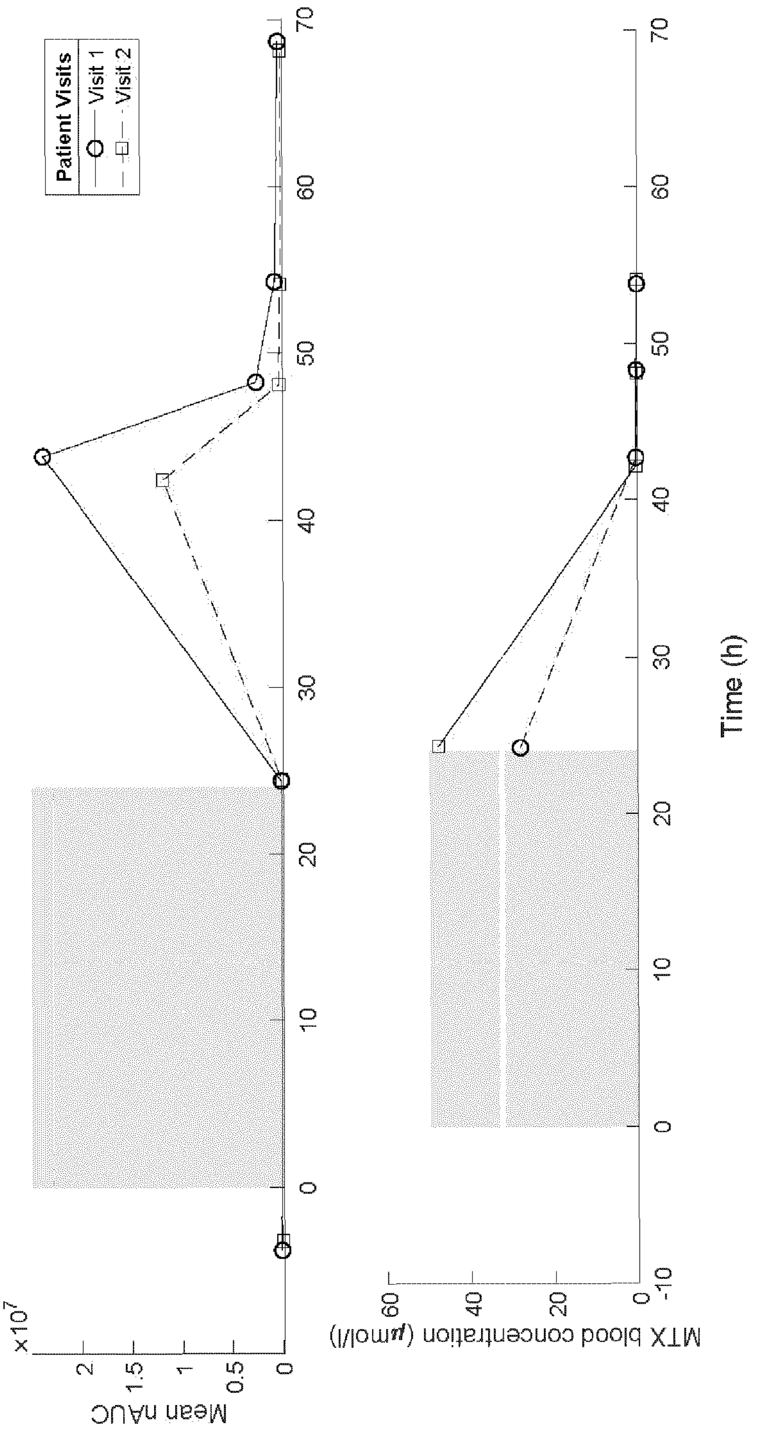
Patient Visits
Visit 1
Visit 2
Mean nAUC
×10^7
0
0.5
1
1.5
2
MTX blood concentration (μmol/l)
60
40
20
0
-10
0
10
20
30
40
50
60
70
Time (h)

Figure 11.

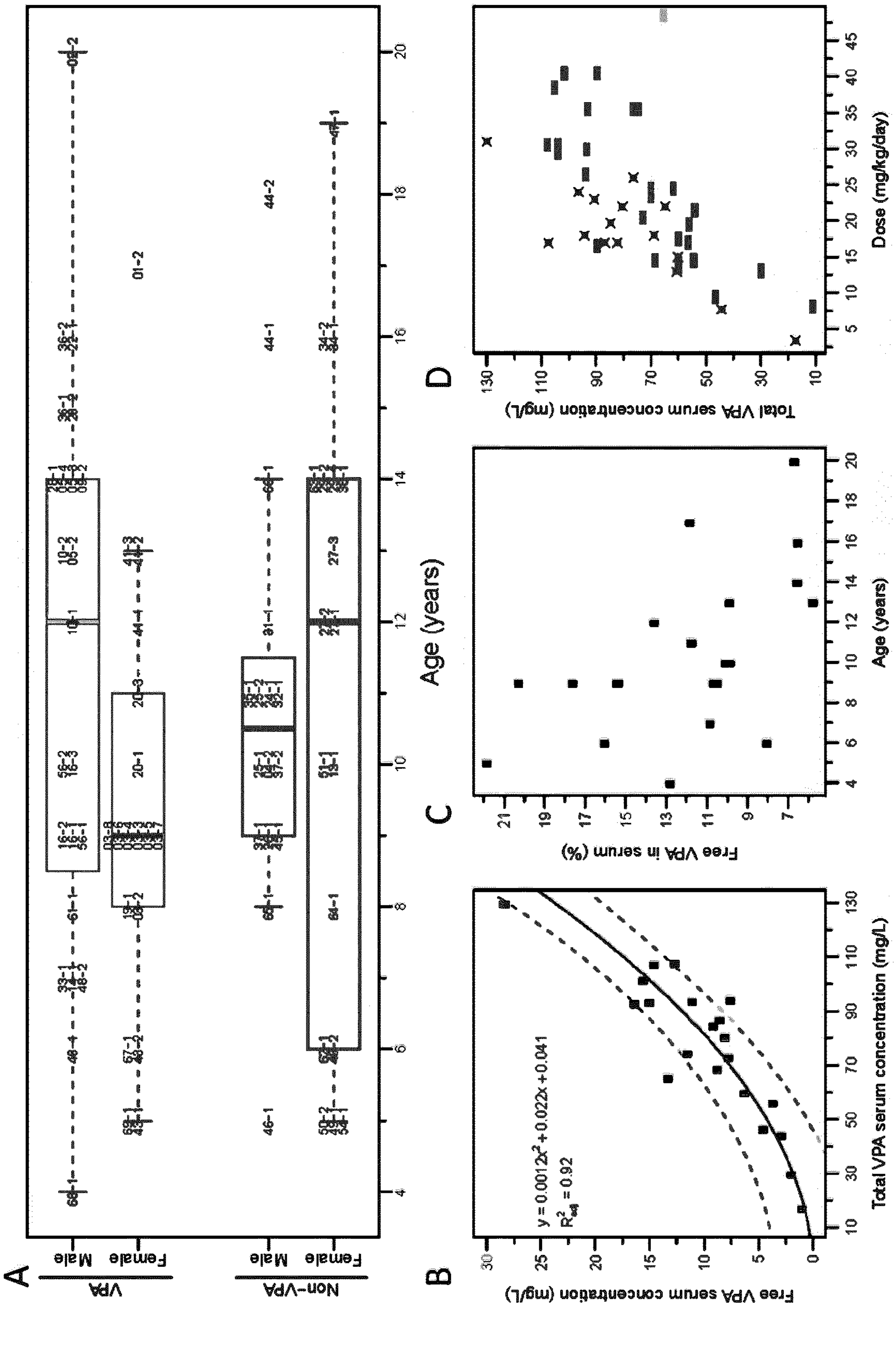
A
VPA
Male
Female
Non-VPA
Male
Female
Age (years)
B
y = 0.0012x^2 + 0.022x + 0.041
R^2_adj = 0.92
Free VPA serum concentration (mg/L)
Total VPA serum concentration (mg/L)
C
Free VPA in serum (%)
Age (years)
D
Total VPA serum concentration (mg/L)
Dose (mg/kg/day)

Figure 12.

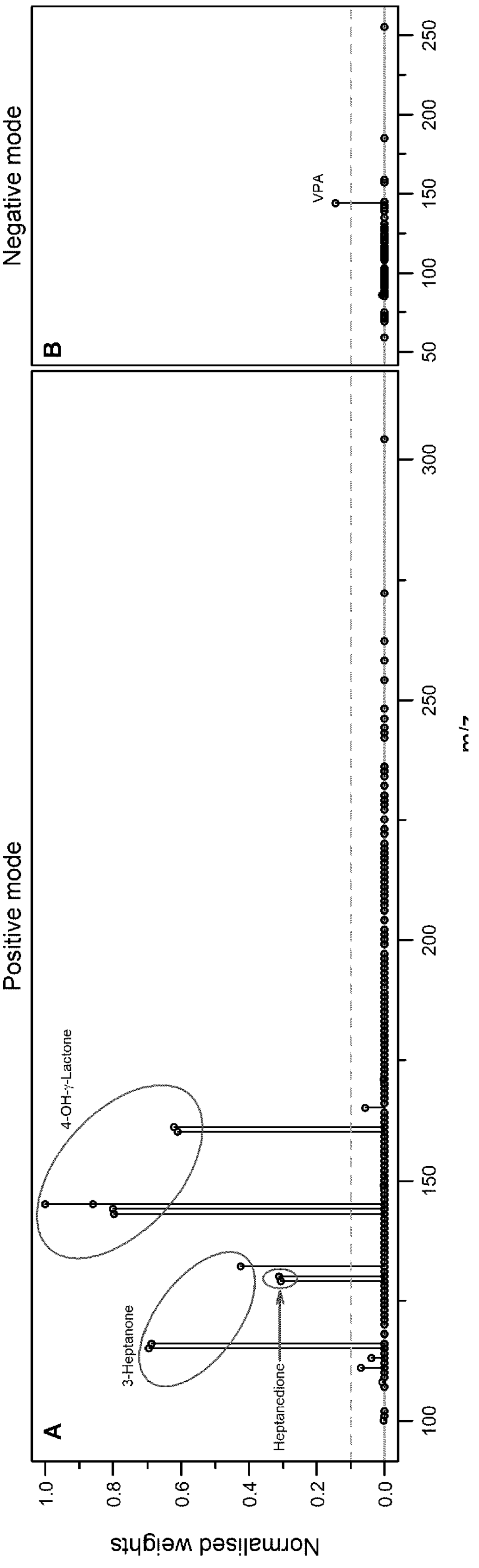
Positive mode
Negative mode
A
B
4-OH-γ-Lactone
3-Heptanone
Heptanedione
VPA
Normalised weights
m/z
0.0
0.2
0.4
0.6
0.8
1.0
100
150
200
250
300
50
100
150
200
250

Figure 13.

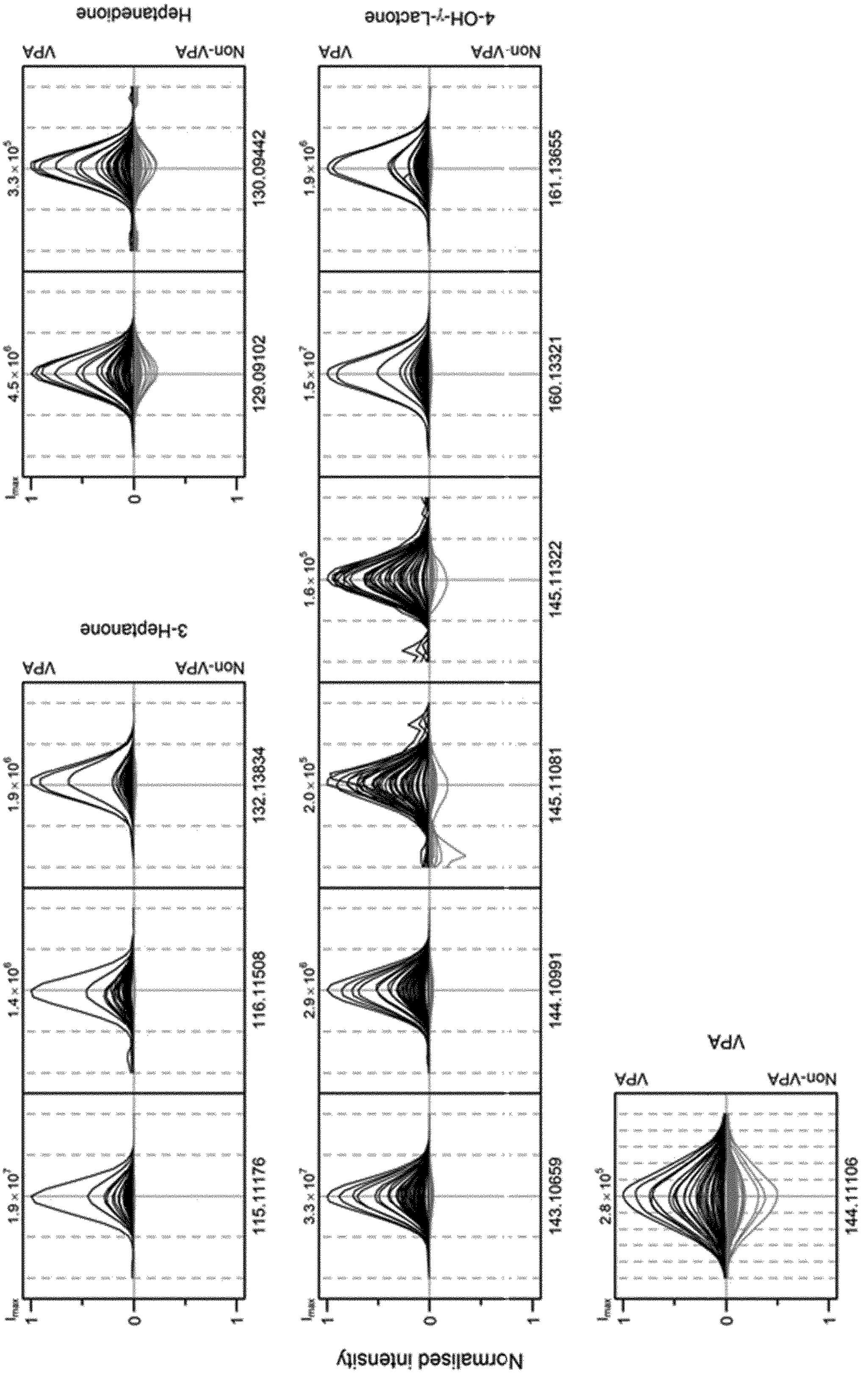
Heptanedione
3-Heptanone
4-OH-γ-Lactone
VPA
Non-VPA
Normalised intensity
115.11176
116.11508
132.13834
129.09102
130.09442
143.10659
144.10991
145.11081
145.11322
160.13321
161.13655
144.11106

Figure 14.

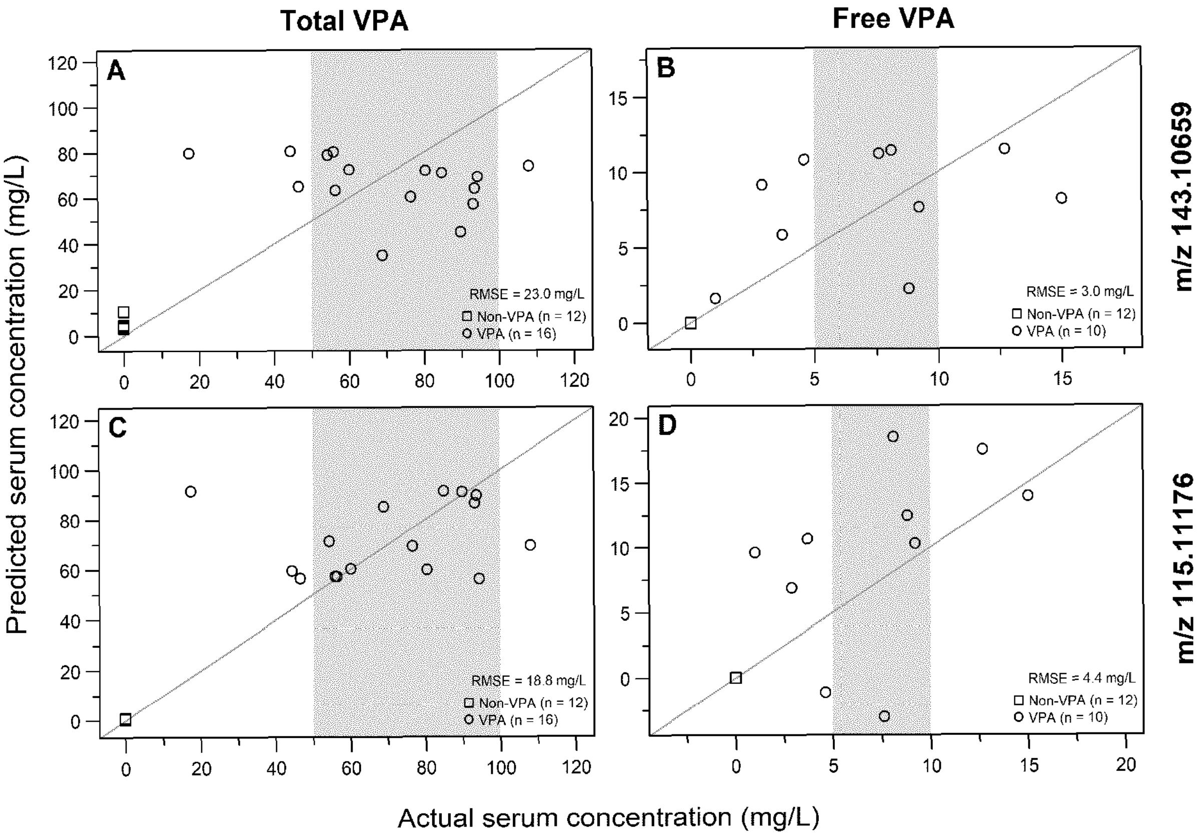
Total VPA
Free VPA
A
B
C
D
RMSE = 23.0 mg/L
RMSE = 3.0 mg/L
RMSE = 18.8 mg/L
RMSE = 4.4 mg/L
Non-VPA (n = 12)
VPA (n = 16)
VPA (n = 10)
m/z 143.10659
m/z 115.11176
Predicted serum concentration (mg/L)
Actual serum concentration (mg/L)

Figure 15.

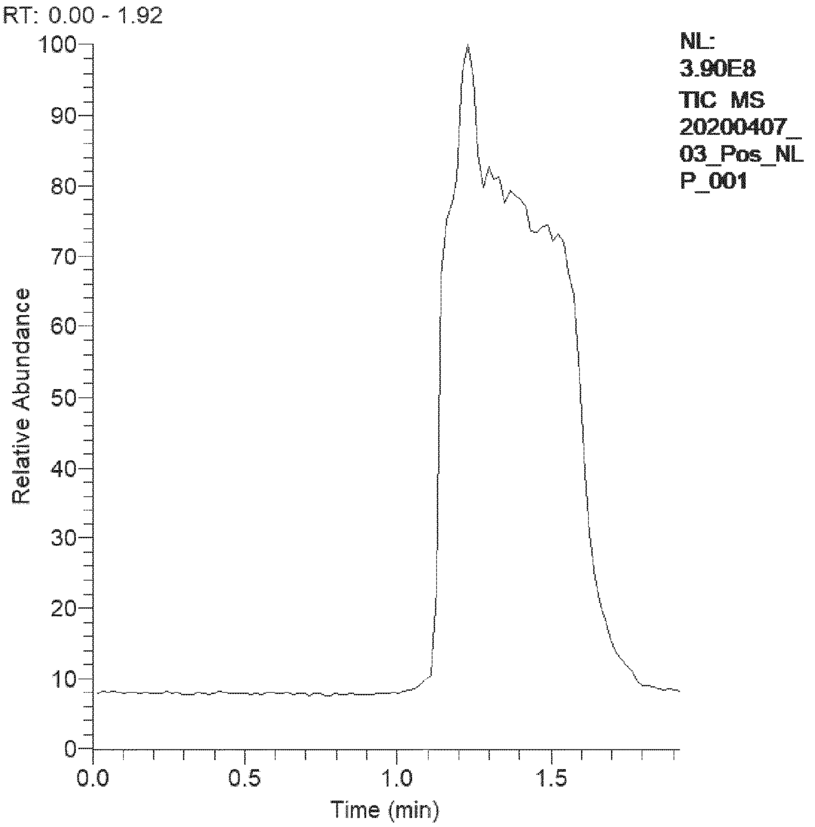


20200407_03_Pos_NLP_001 #74-88 RT: 1.28-1.52 AV: 15 NL: 1.79E7
T: FTMS + p ESI Full lock ms [70.0000-1000.0000]

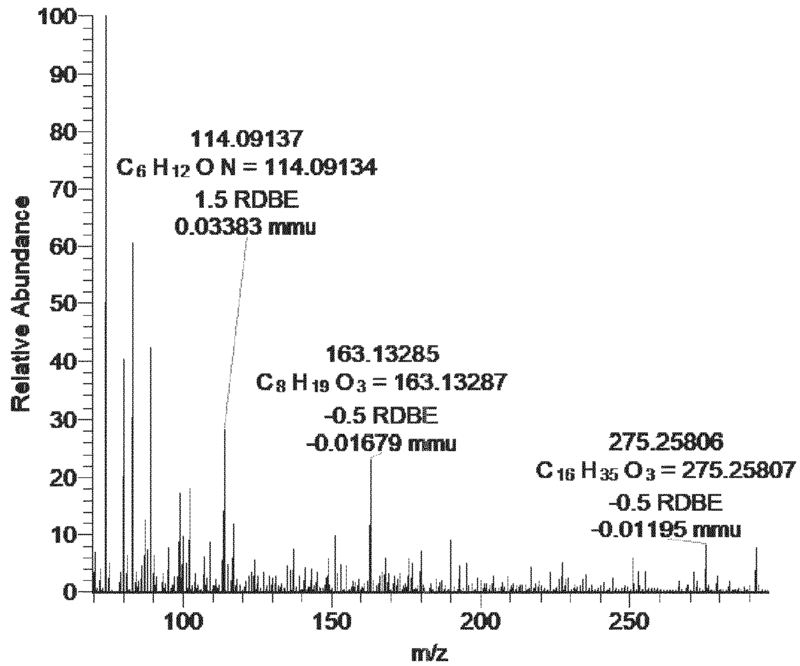


Figure 16.

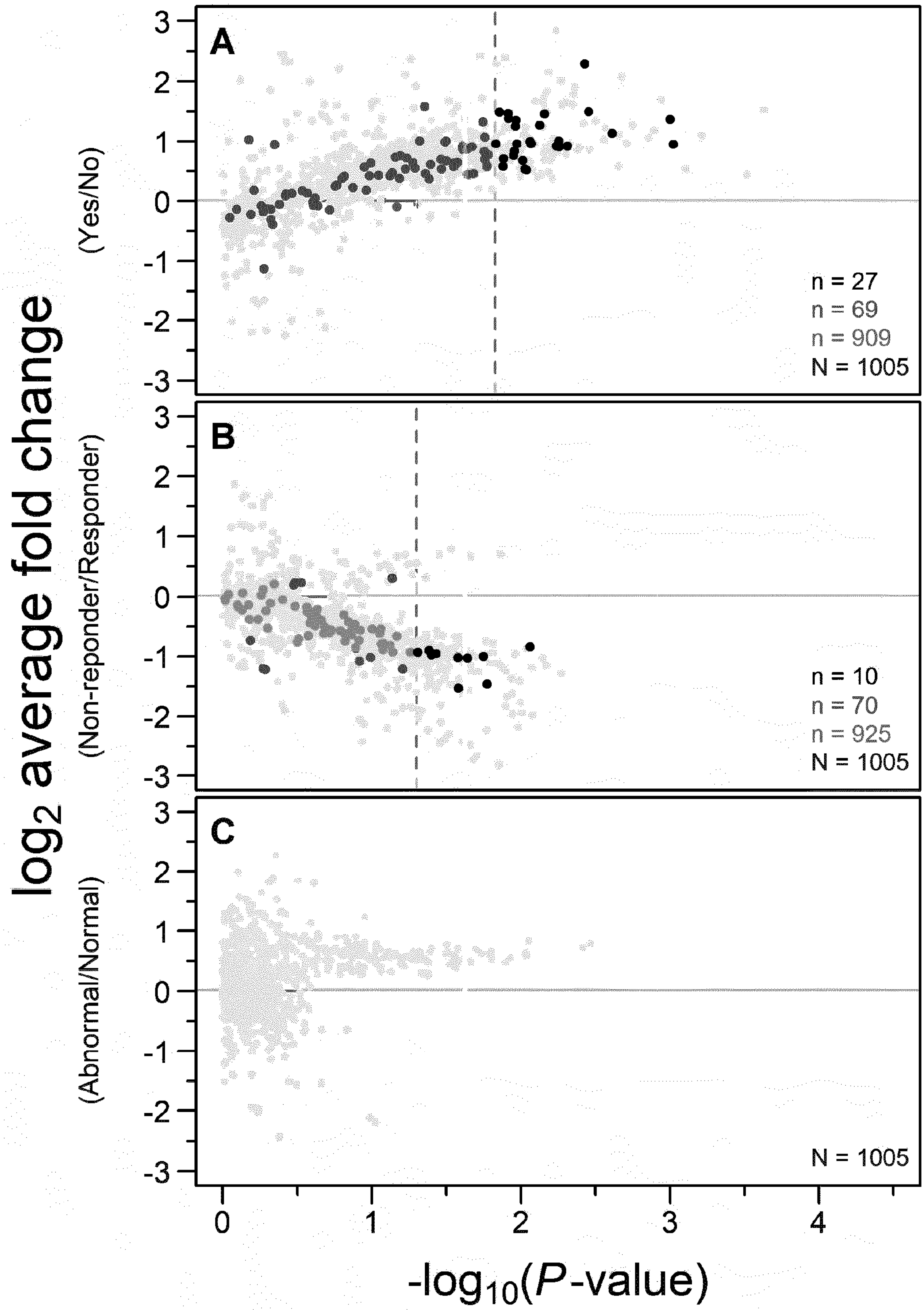


Figure 17 part 1.

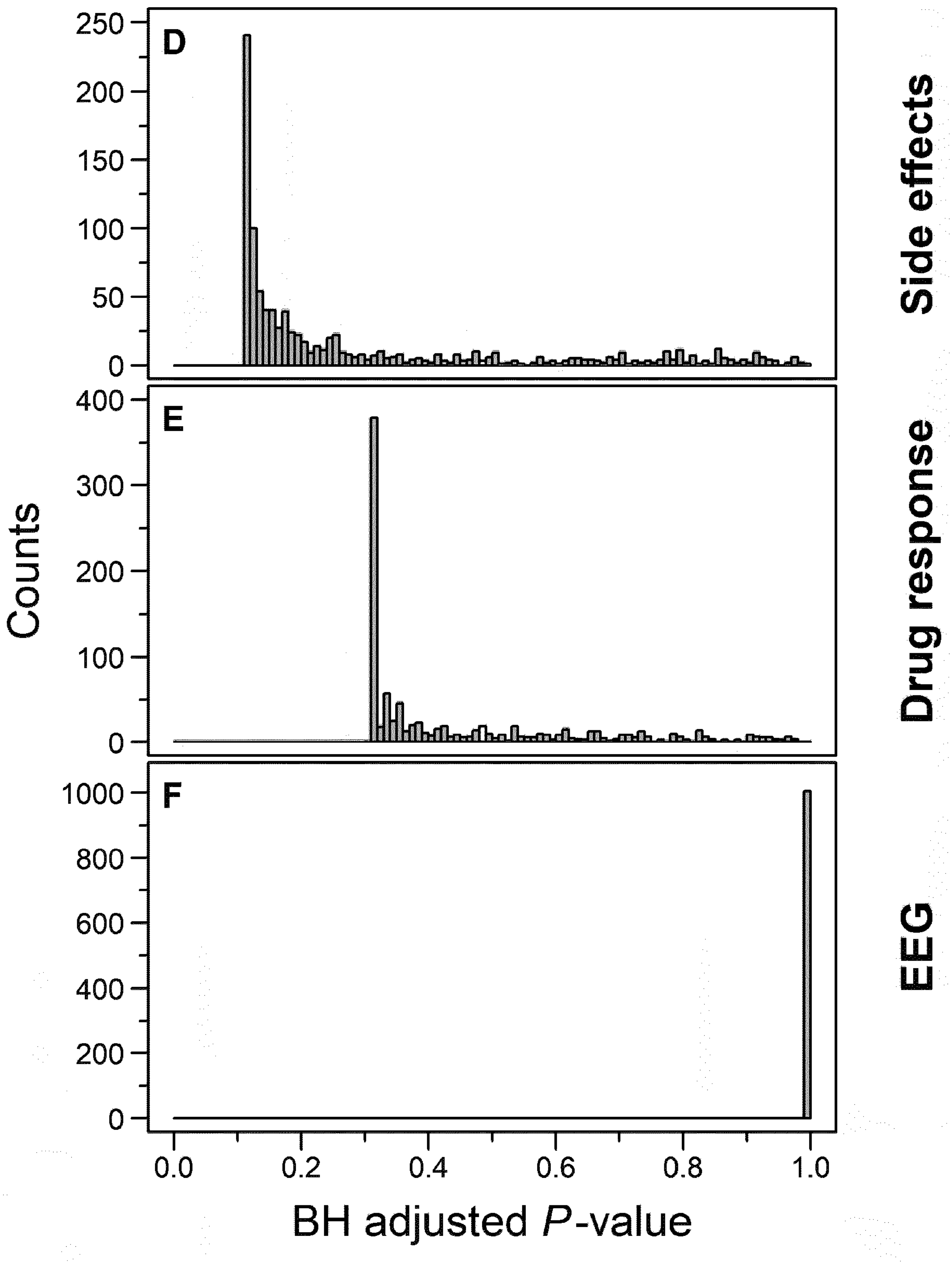


Figure 17 part 2.

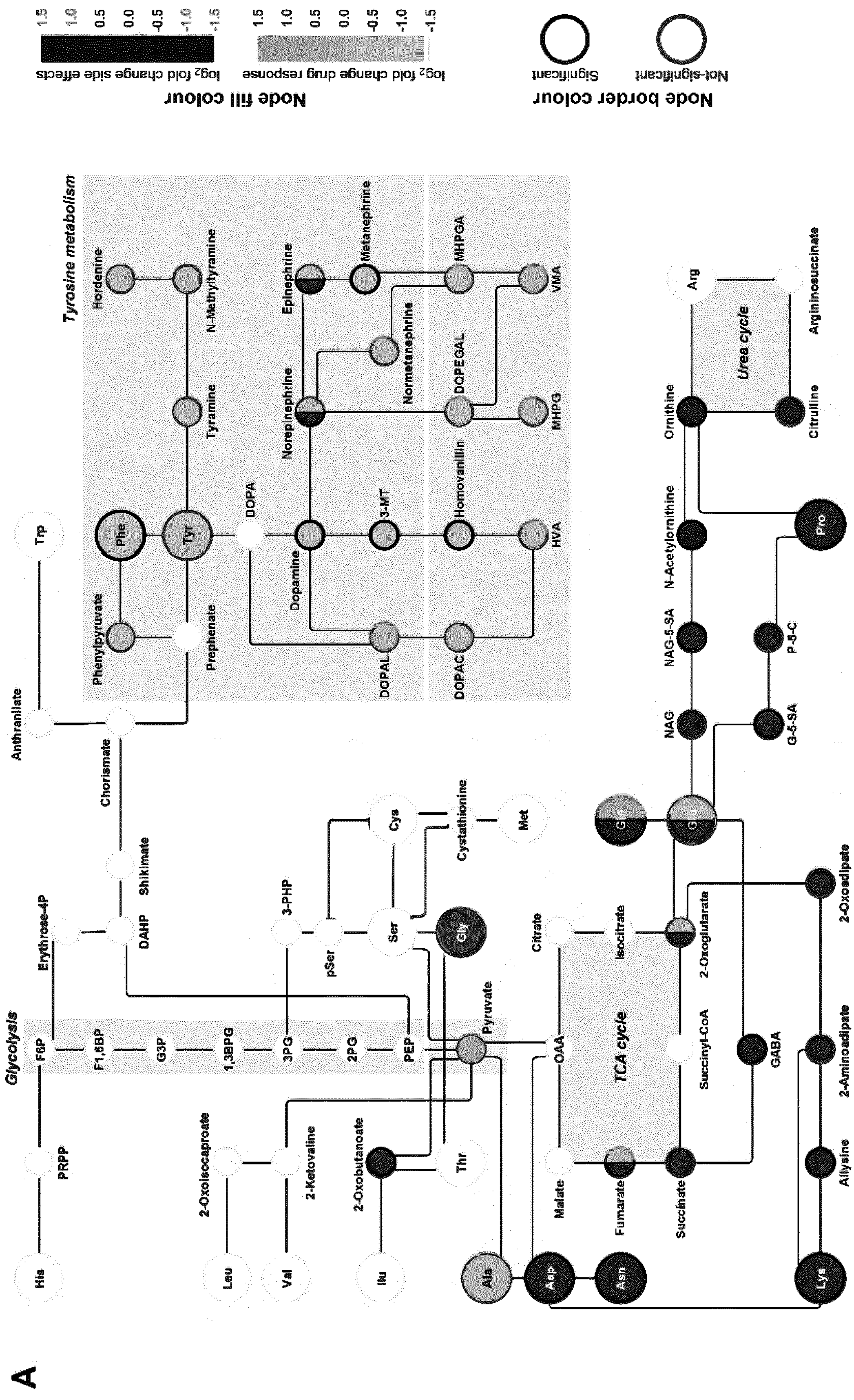


Figure 18 part 1.

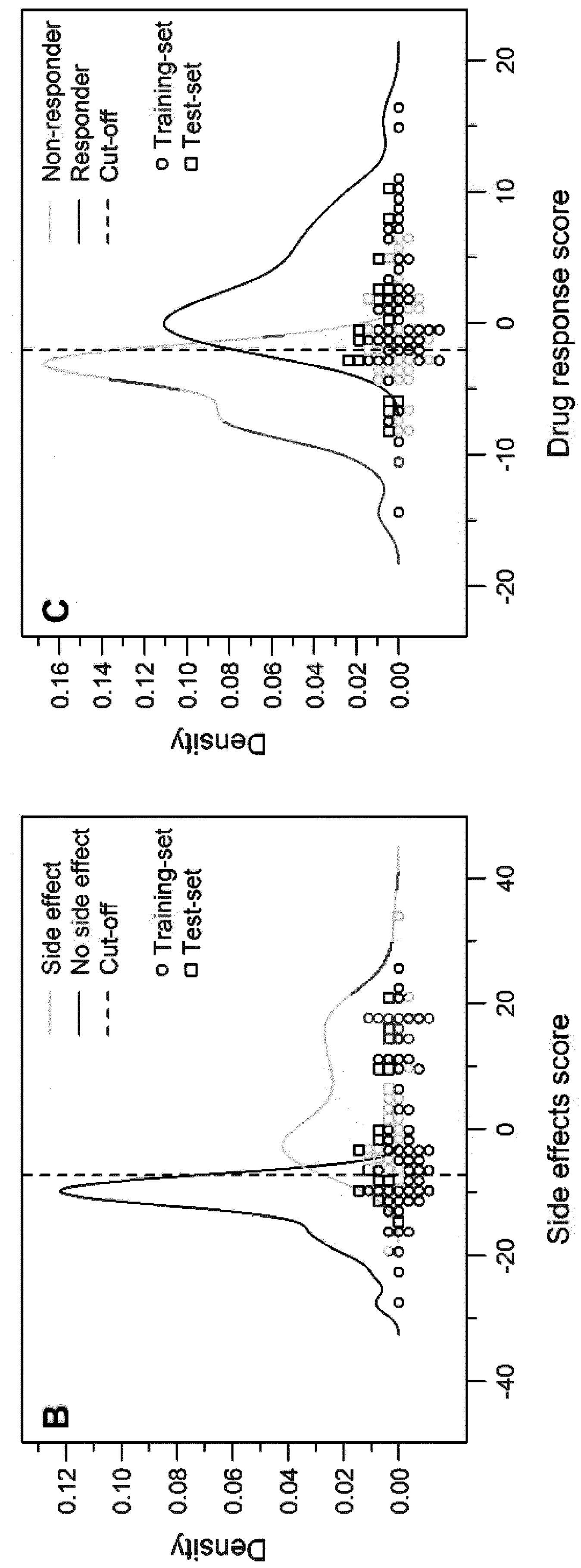


Figure 18 part 2.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20120016252 A1
- US 732770
- US 12556247 B

### Non-patent literature cited in the description


- **BEGHI, E. et al.** Global, regional, and national burden of epilepsy, 1990-2016: a systematic analysis for the Global Burden of Disease Study 2016. *The Lancet Neurology*, 2019, vol. 18 (4), 357-375
- **BREGY, L. et al.** Real-time breath analysis with active capillary plasma ionization-ambient mass spectrometry. *J. Breath Res.*, 2014, vol. 8 (2)
- **CLELLAND, C.L. et al.** Evidence that COMT genotype and proline interact on negative-symptom outcomes in schizophrenia and bipolar disorder.. *Translational psychiatry*, 2016, vol. 6 (9), e891
- Introduction to Therapeutic Drug Monitoring: Frequently and Less Frequently Monitored Drugs. **DASGUPTA, A.** Ther. Drug Monit.. 2012, 1-29
- **DURAN, J. A.** ; **ABADIN, J. A.** ; **SANCHEZ, A.** ; **SERRANO, J. S.** Free Plasma Fraction of Valproic Acid in Epileptic Patients. *Drug Invest*, 1993, vol. 5 (2), 108-113
- **FERNSTROM, J.D.** Aromatic amino acids and monoamine synthesis in the central nervous system: influence of the diet.. *The Journal of Nutritional Biochemistry*, 1990, vol. 1 (10), 508-517
- **KANG, J.** ; **LEE, M.** *Overview of Therapeutic Drug Monitoring.*, 2009, 1-10
- **LÖSCHER, W.** Basic pharmacology of valproate: A review after 35 years of clinical use for the treatment of epilepsy.. *CNS Drugs*, 2002, vol. 16 (10), 669-694
- **NELSON, L.S.** The Shewhart Control Chart-Tests for Special Causes. *Journal of Quality Technology*, October 1984, vol. 16 (4), 238-239
- **SCHILLER, Y.** Seizure relapse and development of drug resistance following long-term seizure remission.. *Archives of Neurology*, 2009, vol. 66 (10), 1233-1239
- **SINGH, K.D. et al.** Standardization procedures for real-time breath analysis by secondary electrospray ionization high-resolution mass spectrometry.. *Analytical and Bioanalytical Chemistry*, 2019
- **SINGH, K.D. et al.** Translating secondary electrospray ionization-high-resolution mass spectrometry to the clinical environment.. *Journal of breath research*, 2018, vol. 12 (2), 027113, http://www.ncbi.nlm.nih.gov/pubmed/29411710
- **TRIPATHI, P.P.** ; **BOZZI, Y.** The role of dopaminergic and serotonergic systems in neurodevelopmental disorders: A focus on epilepsy and seizure susceptibility. *BioImpacts*, 2015, vol. 5 (2), 97-102, http://dx.doi.org/10.15171/bi.2015.07
- **WEAVING G** ; **BATSTONE GF** ; **JONES RG**. Age and sex variation in serum albumin concentration: an observational study.. *Ann Clin Biochem.*, 2016, vol. 53 (1), 106-111